(19) **Europäisches Patentamt / European Patent Office / Office européen des brevets**

(11) **EP 4 691 627 A1**

(12) **EUROPEAN PATENT APPLICATION**
published in accordance with Art. 153(4) EPC

(43) Date of publication:
**11.02.2026 Bulletin 2026/07**

(21) Application number: **24780240.8**

(22) Date of filing: **26.03.2024**

(51) International Patent Classification (IPC):
**B01J 20/26** (2006.01)     **A61F 13/53** (2006.01)
**B01J 20/28** (2006.01)

(52) Cooperative Patent Classification (CPC):
**A61F 13/53; B01J 20/26; B01J 20/28**

(86) International application number:
**PCT/JP2024/011819**

(87) International publication number:
**WO 2024/204126 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.03.2023 JP 2023050601**

(71) Applicant: **Nippon Shokubai Co., Ltd.**
**Osaka-shi, Osaka 541-0043 (JP)**

(72) Inventors:
• **ONISHI, Keita**
  **Himeji-shi, Hyogo 671-1282 (JP)**

• **OGA, Yosuke**
  **Himeji-shi, Hyogo 671-1282 (JP)**
• **SHIMIZU, Ryo**
  **Himeji-shi, Hyogo 671-1282 (JP)**
• **IKEUCHI, Hiroyuki**
  **Himeji-shi, Hyogo 671-1282 (JP)**
• **ENOKIDA, Yusuke**
  **Himeji-shi, Hyogo 671-1282 (JP)**
• **TAMAKI, Mariko**
  **Himeji-shi, Hyogo 671-1282 (JP)**
• **TORII, Kazushi**
  **Himeji-shi, Hyogo 671-1282 (JP)**

(74) Representative: **Hoffmann Eitle**
**Patent- und Rechtsanwälte PartmbB**
**Arabellastraße 30**
**81925 München (DE)**

(54) **PARTICULATE WATER ABSORBENT, ABSORBER CONTAINING SAID PARTICULATE WATER ABSORBENT, AND SANITARY PRODUCT CONTAINING SAID ABSORBER**

(57)     Provided is a particulate water-absorbing agent that can be used for producing a sanitary product capable of further reducing the re-wetting of absorbed liquid in actual use. The subject particulate water-absorbing agent is a particulate water-absorbing agent having a fluid retention capacity of more than 50 g/g when a swollen gel obtained by bringing the particulate water-absorbing agent into contact with a 0.9% aqueous sodium chloride solution for 10 minutes is let stand for 1 minute without pressure, and having a fluid retention capacity of more than 41.5 g/g when the swollen gel is let stand for 1 minute under a pressure of 4.83 kPa.

EP 4 691 627 A1

**Description**

Technical Field

The present invention relates to a particulate water-absorbing agent, an absorbent body containing the particulate water-absorbing agent, and a sanitary product including the absorbent body.

Background Art

A water-absorbent resin (Super Absorbent Polymer: SAP) is a water-swellable, water-insoluble polymer gelling agent. A particulate water-absorbing agent containing a water-absorbent resin as a main component is used in absorbent articles for various applications such as sanitary articles including disposable diapers, sanitary napkins, and adult incontinence products, soil water retaining agents for agriculture and horticulture, and water blocking agents for industrial use. Many monomers and hydrophilic polymers have been proposed as raw materials for such a water-absorbent resin. For the water-absorbent resin, from the viewpoint of performance and cost, a polyacrylic acid (salt)-based water-absorbent resin using acrylic acid and/or a salt thereof as a monomer is most often used.

With the improvement in performance of a disposable diaper, which is the main application of particulate water-absorbing agents, many functions (physical properties) are required for particulate water-absorbing agents. One of the many required functions is a function of having excellent liquid retention capability even when pressure is applied from the outside to particulate water-absorbing agents in a swollen state and reducing re-wet.

An absorbent article such as a sanitary product including a disposable diaper is not replaced every time a liquid to be absorbed such as urine is discharged, and the absorbent article is typically subjected to a plurality of liquid discharges. The particulate water-absorbing agent having absorbed liquid is in a swollen state. Therefore, it is possible for a user to use an absorbent article with a particulate water-absorbing agent in a swollen state. The particulate water-absorbing agent in such a swollen state is required to have a function of having excellent liquid retention capability and reducing re-wet, regardless of the user's daily activities, particularly in a situation where pressure is applied to the particulate water-absorbing agent from the outside, including a situation where the weight of the user is applied to the absorbent article. Examples of such a situation where the weight is applied to the absorbent article include a supine position and a sitting position.

Examples of the particulate water-absorbing agent having the above-described function include a particulate water-absorbing agent described in Patent Document 1. Patent Document 1 proposes a particulate water-absorbing agent, in which AAP (2.06 kPa), absorption capacity under pressure of 2.06 kPa; CRC, absorption capacity without pressure; and RCAP (2.06 kPa), absorption capacity against pressure after swelling have a specific relationship. Here, RCAP (2.06 kPa) represents a weight change, that is fluid retention capacity, of the particulate water-absorbing agent before and after the operation of immersing a particulate water-absorbing agent in a 0.9 wt.% aqueous sodium chloride solution for 1 hour to form a swollen gel and then letting the swollen gel stand under a load of 2.06 kPa for 1 minute.

Citation List

Patent Document

Patent Document 1: WO 2021/201177

Summary of Invention

Technical Problem

However, when sanitary products such as adult diapers in particular are actually in use, the particulate water-absorbing agent contained in the sanitary products needs to absorb a large amount of liquid such as urine in a time shorter than the immersion time (1 hour) for the measurement of RCAP (2.06 kPa) under no pressure. In addition, it is assumed that, when the sanitary products are actually in use, the particulate water-absorbing agent in a swollen state is held under no pressure or subjected to a pressure higher than 2.06 kPa, depending on the motion of the user.

Therefore, RCAP (2.06 kPa) is not necessarily a parameter corresponding to the conditions during actual use of the sanitary product. Therefore, the sanitary product containing the particulate water-absorbing agent described in Patent Document 1 has room for improvement in terms of reduction of re-wetting of the absorbed liquid during actual use.

An object of the present invention is to provide a particulate water-absorbing agent that can be used for producing a sanitary product capable of further reducing the re-wetting of an absorbed liquid during actual use.

Solution to Problem

The present inventors found two types of novel parameters corresponding to the conditions of actual use of the sanitary product and also found that a particulate water-absorbing agent having those parameters within specific ranges can solve the issues and conceived the present invention. The parameters are: a parameter corresponding to a fluid retention capacity when a swollen gel, which is obtained by swelling a particulate water-absorbing agent in a short period of time, is held under no pressure; and a parameter corresponding to a fluid retention capacity when the swollen gel is held under high pressure.

That is, an embodiment of the present invention relates to a particulate water-absorbing agent containing a surface-crosslinked polyacrylic acid (salt)-based water-absorbent resin as a main component, the particulate water-absorbing agent satisfying the following Formula (A) and Formula (B):

$$SRC\ (NP) > 50.0\ g/g\ (A)$$

$$SRC\ (4.83\ kPa) > 41.5\ g/g\ (B)$$

wherein the SRC (NP) is measured by a method including the following steps (a) to (c).

(a) Obtaining a swollen gel by bringing the particulate water-absorbing agent ($W_0$) [g] into contact with a 0.9% aqueous sodium chloride solution (hereinafter referred to as a "test solution") for 10 minutes with no load applied.
(b) Separating the swollen gel obtained in the step (a) and the test solution.
(c) Measuring a weight ($W_1$) [g] of the swollen gel separated in the step (b), and calculating the SRC (NP) of the particulate water-absorbing agent based on the following Formula (1):

$$SRC\ (NP)\ [g/g] = W_1/W_0\ (1)$$

The SRC (4.83 kPa) is measured by the method including the steps (a) to (c) except that that, instead of the step (c), steps (c') to (e') described below are performed.
(c') Applying a load of 4.83 kPa to the swollen gel separated in the step (b) for 1 minute.
(d') Removing an exudate exuded from the swollen gel in the step (c').
(e') Measuring a weight ($W_2$) [g] of the swollen gel obtained in the step (d'), and calculating the SRC (4.83 kPa) based on the following Formula (2):

$$SRC\ (4.83\ kPa)\ [g/g] = W_2/W_0\ (2)$$

Advantageous Effects of Invention

The particulate water-absorbing agent according to an embodiment of the present invention exhibits an effect of being able to be used for production of a sanitary product that can further reduce re-wetting of absorbed liquid during actual use.

Description of Embodiments

Hereinafter, the present invention will be described with reference to the best embodiment thereof. Throughout the present specification, it is to be understood that the expression of a singular form includes also a concept of a plural form thereof, unless otherwise specified. Thus, it should be understood that a singular article (for example, "a", "an", "the", and the like in the case of English) includes a concept of its plural form unless otherwise specified. It should also be understood that the terms used herein are used having the meanings commonly used in the art, unless otherwise specified. Accordingly, unless defined otherwise, all technical and scientific terms used herein have the same meanings as commonly understood by those skilled in the art to which the present invention belongs. In a case where there is any conflict, the present specification, including definitions, will prevail. The present invention is not limited to the following embodiments, and can be variously modified within the scope of the claims.

1. Definition of Terms

(1-1) "Water-Absorbent Resin"

The "water-absorbent resin" as in the present invention refers to a crosslinked polymer having "swellability" and "insolubility", in that the crosslinked polymer swells by absorbing liquid and forms an insoluble gel, and satisfies the following physical properties. That is, the water-absorbent resin refers to the crosslinked polymer satisfying the physical properties of, for "water swellability", CRC of 5 g/g or more as defined in ERT441.2-02, and for "water insolubility", Ext of 50 wt.% or less as defined in ERT470.2-02.

The water-absorbent resin can be designed, as appropriate, depending on the application thereof and is not particularly limited, and is preferably a hydrophilic crosslinked polymer obtained by crosslinking and polymerizing an unsaturated monomer having a carboxyl group. Further, the water-absorbent resin is not limited to a form in which the whole amount (100 wt.%) is a polymer, and may be a water-absorbent resin composition containing additives and the like within a range satisfying the physical properties (CRC, Ext).

Further, the water-absorbent resin in an embodiment of the present invention is not limited to a final product, and may refer to an intermediate in a production process of the water-absorbent resin (e.g., a crosslinked hydrogel polymer after polymerization, a dried polymer after drying, and a water-absorbent resin powder before surface-crosslinking). In the present specification, all of these are collectively referred to as "water-absorbent resin". Examples of the form of the water-absorbent resin include a sheet form, a fibrous form, a film form, a particulate form, and a gel form, and the water-absorbent resin in an embodiment of the present invention is mainly in a particulate form (powder).

(1-2) "Particulate Water-Absorbing Agent"

In the present specification, the water-absorbing agent contains a water-absorbent resin as a main component. In the present specification, the particulate water-absorbing agent means a water-absorbing agent in a particulate form (also referred to as powder form), which contains water-absorbent resin particles as a main component, and it is referred to as a particulate water-absorbing agent regardless of whether it is a single particle of a particulate water-absorbing agent or a plurality of particles of a particulate water-absorbing agent. The term "particulate" means having the form of particles, which refers to a solid or liquid granular small object having a measurable size (Japanese Industrial Standards: JIS, Dictionary of Industrial Terms, 4th edition, p.2002). In the present specification, the particulate water-absorbing agent may be simply referred to as a water-absorbing agent.

In the present specification, an aqueous liquid is not limited to water, and may be urine, blood, sweat, feces, waste liquid, moisture, vapor, ice, a mixture of water and an organic solvent and/or an inorganic solvent, rainwater, groundwater, or the like, and is not particularly limited as long as it contains water. Preferable examples of the aqueous liquid include urine, menstrual blood, sweat, and other body fluids.

The particulate water-absorbing agent according to an embodiment of the present invention is suitably used in a hygienic material for absorbing the aqueous liquid. The particulate water-absorbing agent contains, as a main component, a surface-crosslinked polyacrylic acid (salt)-based water-absorbent resin (particle) (hereinafter, also simply referred to as a polyacrylic acid (salt)-based water-absorbent resin). That is, the surface-crosslinked polyacrylic acid (salt)-based water-absorbent resin is included preferably from 60 to 100 wt.%, from 70 to 100 wt.%, from 80 to 100 wt.%, or from 90 to 100 wt.% in the particulate water-absorbing agent. Additionally, the particulate water-absorbing agent optionally includes one or more materials selected from the group consisting of other water-absorbent resin particles, water, and additives. The additives include inorganic colloidal particles, water-insoluble inorganic particles, a water-soluble polyvalent metal cation-containing compound, or the like. A suitable moisture content of the particulate water-absorbing agent is from 0.2 to 30 wt.%. That is, a water-absorbent resin composition in which these components are integrated is also encompassed in the scope of the particulate water-absorbing agent.

The upper limit of the content of the polyacrylic acid (salt)-based water-absorbent resin in the water-absorbing agent is about 99 wt.%, particularly 95 wt.%, 90 wt.%, or 85 wt.%, and preferably the water-absorbent resin further includes water and/or an additive (inorganic colloidal particles, water-insoluble inorganic particles, or a water-soluble polyvalent metal cation-containing compound) described later.

In addition, the particulate water-absorbing agent according to an embodiment of the present invention contains a polyacrylic acid (salt)-based water-absorbent resin as a main component and may contain other water-absorbent resins. Examples of the other water-absorbent resins include polysulfonic acid (salt)-based water-absorbent resins, maleic anhydride (salt)-based water-absorbent resins, polyacrylamide-based water-absorbent resins, polyvinyl alcohol-based water-absorbent resins, polyethylene oxide-based water-absorbent resins, polyaspartic acid (salt)-based water-absorbent resins, polyglutamic acid (salt)-based water-absorbent resins, polyalginic acid (salt)-based water-absorbent resins, starch-based water-absorbent resins, and cellulose-based polymers. The other water-absorbent resins may be one type or two or more types.

(1-3) "Polyacrylic Acid (Salt)" and "Polyacrylic Acid (Salt)-Based Water-Absorbent Resin"

The term "polyacrylic acid (salt)" in an embodiment of the present invention refers to polyacrylic acid and/or a salt thereof. Note that the "polyacrylic acid (salt)-based water-absorbent resin" is a polyacrylic acid (salt) crosslinked polymer which contains, as a main component, a structural unit derived from acrylic acid and/or a salt thereof (hereinafter, referred to as "acrylic acid (salt)") as a repeating unit and an internal crosslinked structure. The polyacrylic acid (salt)-based water-absorbent resin is preferably surface-crosslinked.

The polyacrylic acid (salt)-based water-absorbent resin is preferably in a particulate form (also referred to as a powder form) in the particulate water-absorbing agent.

The term "main component" means that the amount (content) of acrylic acid (salt) used is typically from 50 to 100 mol%, preferably from 70 to 100 mol%, more preferably from 90 to 100 mol%, and further preferably substantially 100 mol%, based on the total amount of the monomers (excluding an internal crosslinking agent) used for polymerization.

(1-4) "EDANA" and "ERT"

"EDANA" is an acronym for the European Disposables and Nonwovens Associations, and "ERT" is an acronym for EDANA Recommended Test Methods, a European standard (substantially world standard) method for measuring water-absorbent resins. In the present invention, unless otherwise specified, the physical properties of the water-absorbent resin are measured in accordance with the original ERT (revised in 2002/known literature).

(1-4-1) "CRC" (ERT441.2-02)

"CRC" is an acronym for Centrifuge Retention Capacity and means absorption capacity without pressure (may also be referred to as "fluid retention capacity") of a particulate water-absorbing agent or a water-absorbent resin.

Specifically, CRC refers to a fluid retention capacity (unit: g/g) after 0.2 g of a particulate water-absorbing agent or a water-absorbent resin is placed in a non-woven fabric bag, then the bag is immersed in a large excess of a 0.9 wt.% aqueous sodium chloride solution for 30 minutes to allow the bag to freely swell, and then the bag is drained with a centrifuge (250 G).

(1-4-2) "PSD" (ERT420.2-02)

"PSD" is an acronym for Particle Size Distribution and means a particle size distribution of a particulate water-absorbing agent or a water-absorbent resin measured by sieve classification.

The weight-average particle size (D50) and the logarithmic standard deviation ($\sigma\zeta$) of a particle size distribution are measured by the same methods as described in "(3) Mass-Average Particle Size (D50) and Logarithmic Standard Deviation ($\sigma\zeta$) of Particle Diameter Distribution" disclosed in US 7638570.

(1-5) Others

As used herein, "from X to Y" indicating a range means "X or more and Y or less". In addition, unless otherwise noted, "t (ton)", a unit of weight, means "metric ton", and "ppm" means "weight ppm" or "mass ppm". Further, "weight" and "mass", "parts by weight" and "parts by mass", and "wt.%" and "mass%" are treated as synonyms, respectively. In addition, "acid (salt)" means "acid and/or a salt thereof", and "(meth)acrylic" means "acrylic and/or methacrylic".

In addition, "liter" may be denoted as "1" or "L", and "weight%" may be denoted as "wt.%" for convenience. Further, in the case of measuring a trace component, a value equal to or less than the detection limit is denoted as N.D.

(Non Detected).

[2] Particulate Water-Absorbing Agent

The particulate water-absorbing agent according to an embodiment of the present invention (hereinafter referred to as a "particulate water-absorbing agent of the present invention") contains a surface-crosslinked polyacrylic acid (salt)-based water-absorbent resin as a main component and satisfies the following Formula (A) and Formula (B):

$$\text{SRC (NP)} > 50.0 \text{ g/g (A)}$$

$$SRC\ (4.83\ kPa) > 41.5\ g/g\ (B)$$

Herein, the SRC (NP) is measured by a method including the following steps (a) to (c).

(a) Obtaining a swollen gel by bringing the particulate water-absorbing agent ($W_0$) [g] into contact with a 0.9% aqueous sodium chloride solution (hereinafter referred to as a "test solution") for 10 minutes with no load applied.
(b) Separating the swollen gel obtained in the step (a) and the test solution.
(c) Measuring a weight ($W_1$) [g] of the swollen gel separated in the step (b), and calculating the SRC (NP) of the particulate water-absorbing agent based on the following Formula (1):

$$SRC\ (NP)\ [g/g] = W_1/W_0\ (1)$$

The SRC (4.83 kPa) is measured by the method including the steps (a) to (c) except that, instead of the step (c), steps (c') to (e') described below are performed.
(c') Applying a load of 4.83 kPa to the swollen gel separated in the step (b) for 1 minute.
(d') Removing an exudate exuded from the swollen gel in the step (c').
(e') Measuring a weight ($W_2$) [g] of the swollen gel obtained in the step (d'), and calculating the SRC (4.83 kPa) based on the following Formula (2):

$$SRC\ (4.83\ kPa)\ [g/g] = W_2/W_0\ (2)$$

The SRC (NP) is a parameter representing the amount of a liquid retained in the particulate water-absorbing agent when the particulate water-absorbing agent absorbs the liquid in a short period of time and swells, and then is retained without pressure. Note that "SRC (NP)" is an acronym for Short-time Retention Capacity (No Pressure). In addition, the SRC (4.83 kPa) is a parameter representing the amount of a liquid retained in the particulate water-absorbing agent when the particulate water-absorbing agent absorbs the liquid in a short period of time and swells, and then is retained under high pressure.
The particulate water-absorbing agent of the present invention satisfies Formula (A) and Formula (B). Therefore, the particulate water-absorbing agent of the present invention can absorb a large amount of liquid in a short period of time and is excellent in the amount of the liquid retained in both cases of being held in a swollen state without pressure and being held in a swollen state under high pressure. Herein, absorbing a large amount of liquid in a short period of time and being excellent in the amount of liquid retained in cases of being held in a swollen state without pressure and under high pressure are important characteristics to further reduce re-wetting during actual use of a sanitary product. These points are the same as described in the section of "Technical Problem" indicated above. Therefore, the particulate water-absorbing agent of the present invention can be used for producing a sanitary product capable of further reducing re-wetting of a liquid absorbed in actual use.
In the step (a), the particulate water-absorbing agent $W_0$ [g] is brought into contact with a 0.9% aqueous sodium chloride solution (hereinafter referred to as "test solution"), which has been adjusted to a temperature of $23 \pm 1°C$, for 10 minutes with no load applied. Herein, in general, when a load is applied to the particulate water-absorbing agent, the particulate water-absorbing agent becomes limited in the water absorbing capacity. Therefore, "(being) brought into contact for 10 minutes with no load applied" as described above means that the particulate water-absorbing agent is brought into contact with a large excess of the test solution without limiting its water absorbing capacity by the applied load, and the particulate water-absorbing agent is allowed to freely swell by keeping for 10 minutes, the particulate water-absorbing agent sufficiently absorbing the test solution. The expression "(being) brought into contact" as described above means that the particulate water-absorbing agent is brought into contact with a test solution so that the particulate water-absorbing agent can absorb the test solution. Herein, the particulate water-absorbing agent and the test solution may be in contact with each other via a member through which a liquid can pass, such as a nonwoven fabric and a wire mesh. Therefore, in the step (a), the amount of the test solution to be used and conditions such as the shape and size of the measuring apparatus are appropriately adjusted so as not to suppress the free swelling of the particulate water-absorbing agent due to a shortage of the test solution to be used for the 10 minutes.
A container for performing the step (a) is not particularly limited, and examples thereof include a cylinder equipped with a piston. It is preferable that a wire mesh, which a particulate water-absorbing agent and a swollen gel do not permeate through as described later and a test solution and an exudate can flow in or be discharged out of as described later, is attached to the bottom surface of the cylinder. As the container, for example, a cylinder and a piston used in a gel bed permeability test described in US 8269060 can be used.
The specific method for performing the step (a) is not particularly limited. In the case of using the cylinder equipped with a piston, examples of the method include a method including the steps provided as the following (a1) to (a3).

(a1) A step of measuring the total weight of the cylinder and the piston, and setting the value as $W_a$ [g].

(a2) A step of, following the step (a1), weighing out a sample of the particulate water-absorbing agent to be measured to approximately 0.9 g and scattering the sample uniformly on the bottom surface of the cylinder. Herein, the exact weight of the sample is weighed in advance and is denoted by $W_0$ [g].

(a3) A step of, following the step (a2), placing the cylinder in a large excess of a 0.9% aqueous sodium chloride solution as a test solution, and bringing the uniformly scattered sample into contact with the test solution for 10 minutes to provide a swollen gel. Herein, in the step (a3), the sample absorbs the test solution and swells without being subjected to a load, and as a result, a swollen gel is obtained.

The shape and size of the measuring apparatus are not limited. From the viewpoint of improving the accuracy of the measurement of the SRC (NP) and the SRC (4.83 kPa), the apparatus used for the measurement is preferably a circular cylinder having a bottom area of from 19 to 40 cm$^2$. In addition, the basis weight of the particulate water-absorbing agent used in the measurement is preferably from 250 to 350 g/m$^2$. As an example, in conducting the measurement with a cylinder and a piston used in a gel bed permeability test described in US 8269060 as the measuring apparatus, the amount of the particulate water-absorbing agent used is preferably 0.9 g.

In the step (b), the swollen gel obtained in the step (a) is separated from the test solution. That is, this means the completion of the contact between the particulate water-absorbing agent and the test solution performed in the step (a) and the removal of the test solution that is present between the hydrogel particles of the swollen gel obtained in the step (a) and has not been absorbed by the hydrogel particles (hereinafter, also referred to as "excess water"). The method for removing the excess water is not particularly limited. Examples of the method include pulling up the swollen gel obtained in the step (a) from a large excess of the test solution, placing it onto a sieve, and leaving to stand, and draining the solution. The time for the draining is preferably approximately 1 minute. The draining is performed until it reaches a state in which the excess water has been removed from the hydrogel particles. The "state in which the excess water has been removed" means a state in which no water droplet falls from the measuring apparatus for 5 seconds.

When the excess water is removed, the surrounding environment is typically preferably at room temperature (from 20 to 25°C) and under normal pressure (1 atm) in order to match the environment of use of sanitary products such as diapers.

In the step (b), in order to make the thickness of the swollen gel uniform, it is preferable to pull up the swollen gel from the test solution with a piston or the like placed on the swollen gel. Further, it is preferable to drain the swollen gel while the piston or the like is placed on the swollen gel. The load applied by the piston is preferably from 0.22 to 0.28 kPa, more preferably from 0.24 to 0.26 kPa, particularly preferably 0.25 kPa, from the viewpoint of making the thickness of the swollen gel uniform with no excessive load applied to the swollen gel.

In the step (c), the weight of the swollen gel separated in the step (b), that is, the weight of the swollen gel separated from the test solution is measured. The method for measuring the weight of the swollen gel is not particularly limited, and the weight can be measured using a commercially available weight scale. The operations of the steps (a) to (c) can be performed while the particulate water-absorbing agent and the swollen gel are placed in a container consisting of a cylinder, a piston, and the like. In this case, the weight of the container is measured in advance, the total weight of the container and the swollen gel is measured, and the weight of the container is subtracted from the total weight, whereby the weight of the swollen gel can be calculated. In addition, when water droplets of the test solution are on the container, the water droplets are removed by using, for example, Kimwipes or the like, before the weight is measured.

In the step (c), the value of the SRC (NP) is calculated based on the following Formula (1) using the weight $W_0$ [g] of the particulate water-absorbing agent before swelling, which is obtained in the step (a), and the weight $W_1$ [g] of the swollen gel obtained in the step (c).

$$\text{SRC (NP) } [g/g] = W_1/W_0 \ (1)$$

In the step (c'), a load of 4.83 kPa is applied for 1 minute to the swollen gel separated in the step (b), that is, the swollen gel separated from the test solution. The surrounding environment when the load is applied to the swollen gel is also typically preferably room temperature (from 20 to 25°C) and normal pressure (1 atm) in order to match the environment of use of the sanitary product such as a diaper, as in the step (b). The load can be applied by, for example, placing a piston on the swollen gel scattered uniformly on the bottom surface of the cylinder, and placing a weight capable of applying the load of 4.83 kPa to the swollen gel on the piston. Herein, applying the load causes a part of the liquid absorbed by the swollen gel in the step (b) to be exuded from the swollen gel. Hereinafter, the exuded liquid is referred to as an "exudate".

In the step (d'), the exudate exuded from the swollen gel obtained in the step (c') is removed. That is, a load of 4.83 kPa is applied for 1 minute, and then the exudate exuded from the swollen gel is removed. The method for removing the exudate is not particularly limited, and examples thereof include a method for performing the following steps (i) and (ii).

(i) Setting a piston on the swollen gel uniformly scattered on a wire net provided on the bottom surface of a cylinder, and placing the piston on a sieve to drain the solution while the load of 4.83 kPa is placed on the piston.
(ii) Removing the exudate exuded onto the piston by using a dropper.

In the step (e'), the weight of the swollen gel obtained in the step (d'), that is, the weight of the swollen gel after the exudate is removed is measured. The method for measuring the weight of the swollen gel is not particularly limited, and the weight can be measured by a commercially available weight scale. The operations of the steps (a), (b), and (c') to (e') can be performed while the particulate water-absorbing agent and the swollen gel are placed in a container consisting of a cylinder, a piston, a weight, and the like. In this case, the weight of the container is measured previously, the total weight of the container and the swollen gel is measured, and the weight of the container is subtracted from the total weight, whereby the weight of the swollen gel can be calculated. In addition, when water droplets of the test solution and the exudate are on the container, the water droplets on the container are removed using, for example, Kimwipes or the like, before the weight is measured.

In the step (e'), the value of the SRC (4.83 kPa) is calculated based on the following Formula (2) using the weight $W_0$ [g] of the particulate water-absorbing agent before swelling obtained in the step (a) and the weight $W_2$ [g] of the swollen gel obtained in the step (e').

$$\text{SRC (4.83 kPa) [g/g]} = W_2/W_0 \quad (2)$$

In addition, "re-wetting", which is typically used as physical property evaluation of a water-absorbent resin, is evaluated on an absorbent sheet (absorbent body) in which absorbent layer containing a water-absorbent resin (water-absorbing agent), pulp and the like is stacked with a nonwoven fabric or the like, and is not evaluated on the water-absorbent resin (water-absorbing agent) itself. The "re-wetting" may be referred to as a return amount or Re-Wet.

(2-1) SRC (NP)

The method for measuring the SRC (NP) is not particularly limited as long as the method includes the steps (a) to (e) described above. The SRC (NP) can be measured by, for example, the method described in Examples of the present application.

The lower limit of the SRC (NP) is more than 50.0 g/g, preferably more than 54.0 g/g, more preferably more than 56.0 g/g, still more preferably more than 58.0 g/g, and particularly preferably 59.0 g/g or more. The upper limit of the SRC (NP) is not particularly limited and is typically 70.0 g/g or less. The SRC (NP) of the particulate water-absorbing agent of the present invention is a value exceeding the preferable lower limit value, and thus the particulate water-absorbing agent can absorb a larger amount of liquid in a short period of time, and can sufficiently retain the large amount of liquid when retained in a swollen state without pressure. As a result, the sanitary product including the particulate water-absorbing agent of the present invention can further reduce the re-wetting of liquid absorbed during actual use.

(2-2) SRC (4.83 kPa)

The method for measuring the SRC (4.83 kPa) is not particularly limited as long as it includes the steps (a), (b), and (c') to (e') described above. The SRC (4.83 kPa) can be measured by, for example, the method described in Examples of the present application.

The lower limit of the SRC (4.83 kPa) is a value more than 41.5 g/g, is preferably a value more than 42.0 g/g, more preferably is a value more than 42.5 g/g, particularly preferably 43.0 g/g. The upper limit of the SRC (4.83 kPa) is not particularly limited and is typically 50.0 g/g or less. Because the SRC (4.83 kPa) of the particulate water-absorbing agent of the present invention is a value exceeding the preferable lower limit value, the particulate water-absorbing agent can absorb a larger amount of liquid in a short period of time and can sufficiently retain the large amount of liquid when held in a swollen state under high pressure of 4.83 kPa. As a result, the sanitary product including the particulate water-absorbing agent of the present invention can further reduce the re-wetting of liquid absorbed during actual use.

(2-3) CRC (Centrifuge Retention Capacity) (ERT441.2-02)

The term "CRC" is an acronym for Centrifuge Retention Capacity and means the absorption capacity without pressure (may also be referred to as "fluid retention capacity") of a particulate water-absorbing agent or a water-absorbent resin.

Specifically, CRC refers to a fluid retention capacity (unit: g/g) measured after 0.2 g of a particulate water-absorbing agent or a water-absorbent resin is placed in a non-woven fabric bag, then the bag is immersed in a large excess of a 0.9 wt.% aqueous sodium chloride solution for 30 minutes to allow the bag to freely swell, and then the bag is drained with a centrifuge (250 G).

The lower limit of CRC (centrifuge retention capacity) of the particulate water-absorbing agent of the present invention is preferably 35 g/g or more, more preferably 36 g/g or more, further preferably 38 g/g or more, still further preferably 39 g/g or more, particularly preferably 40 g/g or more, and further particularly preferably 41 g/g or more. When CRC is 35 g/g or more, the absorption amount of the particulate water-absorbing agent becomes appropriate, and the performance as an absorbent body in sanitary products such as diapers is ensured. In addition, the upper limit of CRC (centrifuge retention capacity) of the particulate water-absorbing agent of the present invention is preferably 70 g/g or less, and more preferably 60 g/g or less. When CRC exceeds 70 g/g, gel blocking is likely to occur when the particulate water-absorbing agent is swollen. Therefore, when CRC is in an appropriate range, the particulate water-absorbing agent is suitable for use in a paper diaper of a high water absorption speed type or the like. CRC can be controlled by the type and/or amount of the internal crosslinking agent.

(2-4) Water Absorption Speed (Vortex Method)

The water absorption speed (Vortex method) of the particulate water-absorbing agent of the present invention is preferably 40 seconds or less, more preferably 38 seconds or less, further preferably 35 seconds or less, particularly preferably 33 seconds or less, and further particularly preferably 31 seconds or less. Controlling the water absorption speed (vortex method) to the above-described range in addition to improving the SRC (NP) and the SEC (4.83 kPa) to the above-described ranges, the absorption amount in a short period of time is further increased, and the performance when used in sanitary articles such as disposable diapers can be improved.

(2-5) Surface Tension (STR)

Surface tension is the work (free energy) required to increase the surface area of a solid and/or liquid per unit area. The surface tension as used herein refers to the surface tension of an aqueous solution with the particulate water-absorbing agent dispersed in a 0.90 mass% aqueous sodium chloride solution. The surface tension is measured by the following procedure. That is, 50 ml of saline adjusted to 20°C and a sufficiently washed fluorocarbon resin rotator having a 25 mm length are placed in a sufficiently washed 100 ml beaker. Subsequently, the surface tensions of the saline are measured using a surface tensiometer (K11 automatic surface tensiometer available from KRUSS GmbH), and it is confirmed that the values of the surface tensions are from 71 to 75 [mN/m]. Following that, 0.5 g of the particulate water-absorbing agent is put into the beaker containing the saline adjusted to 20°C, the surface tension of which is measured previously, and stirred for four minutes under the condition of 500 rpm. After four minutes, the stirring is stopped, and the particulate water-absorbing agent containing water has settled, and then the surface tension of the supernatant is measured by performing the same operation again. In an embodiment of the present invention, a plate method using a platinum plate is adopted, and the plate is used after being sufficiently washed with deionized water and heated and washed with a gas burner before each measurement.

The surface tension of the particulate water-absorbing agent of the present invention is preferably 66 [mN/m] or more, more preferably 68 [mN/m] or more, further preferably 70 [mN/m] or more, still more preferably 71 [mN/m] or more, and particularly preferably 72 [mN/m] or more. When the surface tension satisfies the above-described conditions, the re-wetting amount in the sanitary product such as a diaper can be further reduced. The upper limit of the surface tension is typically 75 [mN/m].

(2-6) Particle Shape

The particle shape of the water-absorbent resin (powder) is preferably an irregularly crushed shape. Herein, the "irregularly crushed shape" refers to particles having crushed shape which is not constant. Compared to spherical particles obtained by reverse phase suspension polymerization or gas phase polymerization, particles having an irregularly crushed shape are preferable for the following reasons (i) to (iii).

(i) The particles having an irregularly crushed shape have a large surface area and a large contact area with a liquid, and thus have an excellent absorption rate.
(ii) The particles having an irregularly crushed shape have an irregular shape, and thus gaps are easily formed between the particles, and the absorption amount is also increased by the liquid retention capacity due to the gaps formed between the particles.
(iii) The particles having an irregularly crushed shape have an irregular shape, and thus have excellent mixability with hydrophilic fibers such as pulp, and high liquid diffusibility due to gaps between the particles.

The particulate water-absorbing agent according to an embodiment of the present invention is preferably a ground product obtained by aqueous solution polymerization. The particles having an irregularly crushed shape are obtained by pulverizing a gel or a dried product (preferably a dried product) of a crosslinked polymer obtained through aqueous solution polymerization. On the other hand, in the case of not undergoing the pulverizing step, a spherical particle or a granulated product of a spherical particle typically obtained by, for example, reverse phase suspension polymerization or droplet polymerization in which a polymerizable monomer is sprayed to polymerize have no irregularly crushed shape. When the particulate water-absorbing agent has an irregularly crushed shape, the particulate water-absorbing agent is superior to a particulate water-absorbing agent having a high average roundness (for example, a particulate water-absorbing agent in spherical shape) in terms of one or more physical properties selected from the group consisting of a water absorption speed, SRC (NP), and SRC (4.83 kPa). In an embodiment of the present invention, the average roundness of the particulate water-absorbing agent is preferably 0.70 or less, more preferably 0.60 or less, and further preferably 0.55 or less.

The average roundness is calculated by, for example, the following method.

• 100 or more particulate water-absorbing agents are randomly selected, and each particulate water-absorbing agent is photographed with an electron microscope (VE-9800 available from KEYENCE CORPORATION) (magnification 50x) to provide an image of the particulate water-absorbing agent, and the perimeter and the area are calculated for each particle using the attached image analysis software. The area and perimeter are then used to calculate the roundness for each particle using the following formula:

Roundness = $4 \times \pi \times$ area/(perimeter)$^2$ and the average value of the obtained values is calculated as the average roundness.

(2-7) Inorganic Colloidal Particles, Water-Insoluble Inorganic Particles, and Water-Soluble Polyvalent Metal Cation-Containing Compound

The particulate water-absorbing agent according to an embodiment of the present invention preferably further contains at least one selected from the group consisting of inorganic colloidal particles, water-insoluble inorganic particles, and a water-soluble polyvalent metal cation-containing compound, from the viewpoint of inhibiting adhesion between particles of the water-absorbing agent.

When the particulate water-absorbing agent of the present invention contains any one of the inorganic colloidal particles, water-insoluble inorganic particles, and water-soluble polyvalent metal cation-containing compound, the particulate water-absorbing agent is excellent in handleability in a humid environment. Therefore, when producing a thin absorbent body for a sanitary product, using such a particulate water-absorbing agent can reduce the likelihood of issues such as the occurrence of aggregation and clogging in transfer pipe lines of a production plant and the inability to be mixed with hydrophilic fibers uniformly. Using such a particulate water-absorbing agent is preferable because a decrease in the performance of a sanitary product can be suppressed when the thin absorbent body containing the particulate water-absorbing agent of the present invention is produced.

The particulate water-absorbing agent can further improve the performance of the particulate water-absorbing agent by including the inorganic colloidal particles. The inorganic colloidal particles mean inorganic particles dispersed in a dispersion medium in a colloidal solution. Specific examples of the inorganic colloidal particles include colloidal silica in which silicon dioxide particles are dispersed in water, and alumina sol in which aluminum oxide is dispersed in water. The content of the inorganic colloidal particles is preferably from 0.001 to 5 parts by weight in terms of solids content, more preferably from 0.01 to 1 part by weight in terms of solids content, per 100 parts by weight of the polyacrylic acid (salt)-based water-absorbent resin in the particulate water-absorbing agent of the present invention.

The particulate water-absorbing agent can further improve the performance of the particulate water-absorbing agent by including the water-insoluble inorganic particles.

The water-insoluble inorganic particles are not particularly limited. Specific examples of the water-insoluble inorganic particles include silicon dioxide (silica), aluminum hydroxide, zinc oxide, talc, zeolite, hydrotalcite, and tricalcium phosphate.

The content of the water-insoluble inorganic particles is preferably 0.01 wt.% or more and less than 10 wt.%, more preferably from 0.1 to 5 wt.%, based on 100 wt.% of the polyacrylic acid (salt)-based water-absorbent resin.

Including a water-soluble polyvalent metal cation-containing compound in the particulate water-absorbing agent of the present invention allows the performance of the particulate water-absorbing agent to be improved.

The water-soluble polyvalent metal cation-containing compound is a compound that contains a divalent or higher, preferably trivalent or higher, metal cation and is water-soluble. The water-soluble polyvalent metal cation compound is not particularly limited. Specific examples of the water-soluble polyvalent metal cation compound include aluminum chloride, potassium zirconium carbonate, zirconium sulfate, aluminum sulfate, potassium aluminum sulfate, and sodium aluminum sulfate.

In the present specification, being water-soluble means a property of being soluble (or easily soluble) in water at room temperature (from 20 to 25°C) and under normal pressure (1 atm), and for example it means a property of having a solubility of 1 g or more in 100 ml of water at room temperature and under normal pressure. In addition, in the present specification, being water-insoluble means a property of being insoluble (or poorly soluble) in water at room temperature (from 20 to 25°C) and under normal pressure (1 atm), and for example it means a property of having a solubility of less than 1 g in 100 ml of water at room temperature and under normal pressure. The water-insolubility is preferably such that the solubility in 100 ml of water at room temperature and under normal pressure is less than 0.1 g.

The content of the water-soluble polyvalent metal cation-containing compound is preferably from 0.001 to 5 parts by weight, more preferably from 0.01 to 2 parts by weight, and further preferably from 0.01 to 1 part by weight in terms of the amount of the polyvalent metal cation, per 100 parts by weight of the polyacrylic acid (salt)-based water-absorbent resin. When the content is within these ranges, the performance of the particulate water-absorbing agent can be improved.

(2-8) Chelating Agent

The particulate water-absorbing agent of the present invention preferably further contains at least one chelating agent for the purpose of reducing coloration and deterioration. The chelating agent may be at least one chelating agent selected from the group consisting of an organic phosphorus-based chelating agent and an aminocarboxylic acid-based chelating agent. The particulate water-absorbing agent that has absorbed urine is deteriorated over time by a trace amount of metal ions and L-ascorbic acid contained in the urine, and thus the liquid retention capacity is lowered, causing a risk that the liquid that has been absorbed once is discharged again from the sanitary article. Containing a chelating agent in the particulate water-absorbing agent makes it possible to suppress re-wetting over time when the particulate water-absorbing agent is used in a sanitary article.

The content of the chelating agent is preferably from 0 to 3 parts by weight, more preferably from 0.005 to 1 parts by weight, and further preferably from 0.01 to 0.5 part by weight, per 100 parts by weight of the polyacrylic acid (salt)-based water-absorbent resin.

In addition, for the chelating agent, for example, the compounds and the amounts thereof used as disclosed under "[2] Chelating Agent" in WO 2011/040530 may be applied to an embodiment of the present invention.

(3) Method for Producing Particulate Water-Absorbing Agent

The production steps (3-1) to (3-8) for the particulate water-absorbing agent according to an embodiment of the present invention will be described below.

(3-1) Preparation Step of Aqueous Monomer Solution

This step is a step of preparing an aqueous solution containing acrylic acid (salt) as a monomer as a main component (hereinafter referred to as "aqueous monomer solution"). A monomeric slurry liquid may be used as long as the water absorption performance of the resulting water-absorbent resin does not deteriorate. However, in this section, an aqueous monomer solution will be described for convenience.

In addition, the "main component" means that the amount (content) of acrylic acid (salt) used is typically 50 mol% or more, preferably 70 mol% or more, and more preferably 90 mol% or more (upper limit: 100 mol%), per the whole monomers (excluding the internal crosslinking agent) to be subjected to the polymerization reaction of the water-absorbent resin.

Acrylic Acid

In an embodiment of the present invention, from the viewpoint of the physical properties and productivity of the resulting particulate water-absorbing agent, acrylic acid and/or a salt thereof (hereinafter referred to as "acrylic acid (salt)") is used as a monomer.

The "acrylic acid" may be a known acrylic acid. The "acrylic acid" may contain, as a polymerization inhibitor, preferably methoxyphenols, more preferably p-methoxyphenol in an amount of preferably 200 ppm or less, more preferably from 10 to 160 ppm, and further preferably from 20 to 100 ppm. The content of the polymerization inhibitor is preferable from the viewpoint of the polymerizability of acrylic acid and/or the color tone of the particulate water-absorbing agent. Regarding impurities in acrylic acid, the compounds disclosed in US 2008/0161512 A are also applied to an embodiment of the present invention.

In addition, the "acrylic acid salt" is a product produced by neutralizing the acrylic acid with the following basic composition. The acrylic acid salt may be a commercially available acrylic acid salt (for example, sodium acrylate), or may be one obtained by neutralizing acrylic acid in a production plant for the particulate water-absorbing agent.

Basic Composition

In an embodiment of the present invention, the "basic composition" refers to a composition containing a basic compound, such as a commercially available aqueous sodium hydroxide solution.

Specific examples of the basic compound include a carbonate or a hydrogen carbonate of an alkali metal, a hydroxide of an alkali metal, ammonia, and an organic amine. Among these, the basic compound is desirably strongly basic from the viewpoint of the physical properties of the particulate water-absorbing agent to be obtained. As the basic compound, for example, hydroxides of alkali metals such as sodium hydroxide, potassium hydroxide, and lithium hydroxide are preferable, and sodium hydroxide is more preferable.

Neutralization

As the neutralization in an embodiment of the present invention, either the neutralization of acrylic acid (before polymerization) or the neutralization of a crosslinked hydrogel polymer obtained by crosslinking polymerization of acrylic acid (after polymerization) (hereinafter referred to as "post-neutralization") can be selected, or both can be used in combination. In addition, these neutralizations may be performed continuously or batchwise, and are not particularly limited, but are preferably performed continuously from the viewpoint of production efficiency and the like.

Regarding conditions such as the apparatus for performing neutralization, the neutralization temperature, and the residence time, the conditions disclosed in WO 2009/123197, US 2008/0194863 A are applied to an embodiment of the present invention.

The rate of neutralization in an embodiment of the present invention is preferably from 10 to 90 mol%, more preferably from 40 to 85 mol%, further preferably from 50 to 80 mol%, and particularly preferably from 60 to 75 mol%, per an acid group of the monomer. When the rate of neutralization is less than 10 mol%, the fluid retention capacity may be significantly reduced. In contrast, when the rate of neutralization is more than 90 mol%, a water-absorbent resin having a high absorption capacity under pressure may not be obtained.

The rate of neutralization is the same even in the case of the post-neutralization. In addition, the above-described rate of neutralization is applied to the rate of neutralization of the particulate water-absorbing agent as a final product. For example, the "rate of neutralization of 75 mol%" means a mixture of 25 mol% of acrylic acid and 75 mol% of an acrylic acid salt. In addition, the mixture may be referred to as a partially neutralized acrylic acid.

Other Monomers

In an embodiment of the present invention, the "other monomers" refer to monomers other than the acrylic acid (salt), and the particulate water-absorbing agent can be produced by using the other monomers in combination with the acrylic acid (salt).

Examples of the other monomers include water-soluble or hydrophobic unsaturated monomers. Specifically, the compounds disclosed in US 2005/0215734 A (excluding acrylic acid) are also applied to an embodiment of the present invention.

Internal Crosslinking Agent

As the internal crosslinking agent used in an embodiment of the present invention, the compounds disclosed in US 6241928 are also applied to an embodiment of the present invention. Among these, one or more compounds are selected in consideration of reactivity. Depending on the form of polymerization and/or the type of the polymerization initiator described below, a crosslinked polymer may be obtained without using an internal crosslinking agent, but it is preferable to use at least one internal crosslinking agent.

The amount of the internal crosslinking agent used is preferably from 0.0001 to 10 mol%, more preferably from 0.001 to 1 mol%, further preferably from 0.001 to 0.5 mol%, still more preferably from 0.005 to 0.1 mol%, particularly preferably from 0.005 to 0.05 mol%, per the total amount of the monomers. When the above-described amount used is within the above-described range, a desired water-absorbent resin is obtained. When the above-described amount used is too small, the gel strength of the resulting water-absorbent resin tends to decrease and the water-soluble content tends to increase. When the above-described amount used is too large, the fluid retention capacity of the resulting water-absorbent resin tends to decrease. The mol% of the whole monomer is a percentage of the number of moles of the internal crosslinking agent to the total number of moles of monomers contained in the aqueous monomer solution.

In an embodiment of the present invention, a method is preferably applied in which a predetermined amount of an internal crosslinking agent is added previously to an aqueous monomer solution, and crosslinking reaction is performed simultaneously with polymerization. In addition to this method, a method for adding an internal crosslinking agent during and/or after polymerization and then crosslinking; a method for performing radical crosslinking using a radical polymerization initiator; a method for performing radiation crosslinking using an active energy ray such as an electron beam or an ultraviolet ray or the like can also be adopted. These methods may be used in combination.

Other Substances Added to Aqueous Monomer Solution

In an embodiment of the present invention, from the viewpoint of improving the physical properties of the resulting water-absorbent resin, the following substances may be added during the preparation of the aqueous monomer solution.
Examples of the substance include hydrophilic polymers such as starch, starch derivatives, cellulose, cellulose derivatives, polyvinyl alcohol, polyacrylic acid (salt), and crosslinked polyacrylic acid (salt). The hydrophilic polymer can be added to the aqueous monomer solution in an amount of preferably 50 wt.% or less, more preferably 20 wt.% or less, further preferably 10 wt.% or less, and particularly preferably 5 wt.% or less (the lower limit is 0 wt.%). In addition, a foaming agent such as a carbonate, an azo compound, and air bubbles, a surfactant, a chelating agent, an $\alpha$-hydroxycarboxylic acid (salt), a chain transfer agent, or the like can be added to the aqueous monomer solution in an amount of preferably 5 wt.% or less, more preferably 1 wt.% or less, and further preferably 0.5 wt.% or less. The lower limit of the amount of the substance added is 0 wt.%. Examples of the chelating agent include diethylenetriaminepentaacetic acid (salt), triethylenetetramine hexaacetic acid (salt), hydroxyethylidenediphosphonic acid (salt), and ethylenediaminetetramethylenephosphonic acid (salt). In addition, as the $\alpha$-hydroxycarboxylic acid (salt), a compound exemplified in an additive addition step described in (3-7) below can be added in the same manner.
The above-described substance may be added not only in the form of being added to the aqueous monomer solution, but also in the form of being added during polymerization, and both of these forms may be used in combination.
When a water-soluble resin or a water-absorbent resin is used as the hydrophilic polymer, a graft polymer, or a water-absorbent resin composition (for example, starch-acrylic acid polymer, PVA-acrylic acid polymer, and the like) is produced. These polymers and water-absorbent resin compositions are also within the scope of the present invention.

Concentration of Monomer Component

In this step, each of the substances described above is added when the aqueous monomer solution is prepared. The concentration of the monomer component in the aqueous monomer solution is not particularly limited, and is preferably from 10 to 80 wt.%, more preferably from 20 to 75 wt.%, and further preferably from 30 to 70 wt.%, from the viewpoint of the physical properties of the water-absorbent resin.
In addition, when aqueous solution polymerization or reverse phase suspension polymerization is adopted, a solvent other than water can be used in combination, as necessary. In this case, the type of the solvent is not particularly limited.
The "concentration of the monomer component" is a value determined by the following Formula (3), and the weights of the graft component, the water-absorbent resin, and the hydrophobic solvent in the reverse phase suspension polymerization are not included in the weight of the aqueous monomer solution.

(Concentration of monomer component (wt.%) = (Weight of monomer component)/(Weight of aqueous monomer solution) $\times$ 100      Formula (3)

(3-2) Polymerization Step

The polymerization step is a step of polymerizing an acrylic acid (salt)-based aqueous monomer solution obtained in the preparation step of an aqueous monomer solution to provide a crosslinked hydrogel polymer (hereinafter, referred to as a "hydrogel").

Polymerization Initiator

Polymerization initiator usable in an embodiment of the present invention is appropriately selected in accordance with a form of polymerization and the like and is not particularly limited to any particular one. Examples thereof include a thermally decomposable polymerization initiator, a photodecomposable polymerization initiator, or a redox-based polymerization initiator in which a reducing agent that accelerates the decomposition of these polymerization initiators is used in combination. Specifically, one or more of the polymerization initiators disclosed in US 7265190 are used. From the viewpoint of the handleability of the polymerization initiator and the physical properties of the particulate water-absorbing agent or the water-absorbent resin, a peroxide or an azo compound is preferably used, a peroxide is more preferably used, and a persulfate is further preferably used.
The amount of the polymerization initiator used is preferably from 0.001 to 1 mol%, more preferably from 0.001 to 0.5 mol%, per monomer. The amount of the reducing agent used is preferably from 0.0001 to 0.02 mol%, per monomer.

Instead of using the polymerization initiator, the polymerization reaction may be performed by irradiating active energy rays such as radiation, an electron beam, and an ultraviolet ray, or these active energy rays and the polymerization initiator may be used in combination.

Form of Polymerization

The form of polymerization applied to an embodiment of the present invention is not particularly limited, but from the viewpoints of water absorption properties, ease of polymerization control, and the like, preferable examples thereof include spray droplet polymerization, aqueous solution polymerization, and reverse phase suspension polymerization, more preferable examples thereof include aqueous solution polymerization and reverse phase suspension polymerization, and further preferable examples thereof include aqueous solution polymerization. Among these, continuous aqueous solution polymerization is particularly preferable. As the continuous aqueous solution polymerization, either continuous belt polymerization or continuous kneader polymerization can be applied.

As specific forms of polymerization, continuous belt polymerization is disclosed in US 4893999, US 6241928, US 2005/215734, and the like, and continuous kneader polymerization is disclosed in US 6987151, US 6710141, and the like. Adopting these forms of the continuous aqueous solution polymerization improves the production efficiency of the water-absorbent resin.

Preferable forms of the continuous aqueous solution polymerization include "high-temperature initiation polymerization" and "high-concentration polymerization". The "high-temperature initiation polymerization" refers to a form in which polymerization is initiated with the temperature of an aqueous monomer solution set to preferably 30°C or more, more preferably 35°C or more, further preferably 40°C or more, and particularly preferably 50°C or more (upper limit is boiling point). The "high-concentration polymerization" refers to a form in which polymerization is performed with a monomer concentration set to preferably 30 wt.% or more, more preferably 35 wt.% or more, further preferably 40 wt.% or more, and particularly preferably 45 wt.% or more (upper limit is saturation concentration). These forms of polymerization can also be used in combination.

Note that in an embodiment of the present invention, polymerization can be performed in an air atmosphere, but from the viewpoint of the color of the resulting water-absorbent resin, polymerization may be performed in an inert gas atmosphere such as nitrogen or argon. In this case, for example, the oxygen concentration is preferably controlled to 1 vol% or less. Dissolved oxygen in an aqueous monomer solution is also preferably substituted with an inert gas (e.g., dissolved oxygen: less than 1 mg/l).

Note that, in an embodiment of the present invention, foaming polymerization can be employed, in which polymerization is performed with bubbles (particularly the inert gas or the like) dispersed in an aqueous monomer solution.

(3-3) Gel-Grinding Step

This step is a step of subjecting the hydrogel obtained in the polymerization step to gel-grinding with, for example, a gel-grinder such as a kneader, a screw extruder such as a meat chopper, or a cutter mill to obtain a particulate hydrogel (hereinafter, referred to as "particulate hydrogel"). Note that, when kneader polymerization is employed in the polymerization step, the polymerization step and the gel-grinding step are performed simultaneously. In addition, when gas phase polymerization or reverse phase suspension polymerization are employed in the polymerization step and a particulate hydrogel is directly obtained through the polymerization process, the gel-grinding step may not be performed.

Controlling the means for the gel-grinding (kneader or the like) and the gel-grinding energy (GGE), which is one of the gel-grinding conditions to be described later allows control of SRC (NP) and SRC (4.83 kPa) of the particulate water-absorbing agent to be produced. In this case, for the conditions and forms of gel-grinding other than the gel-grinding energy (GGE), the contents disclosed in WO 2011/126079 A are preferably applied to an embodiment of the present invention.

(3-4) Drying Step

This step is a step of obtaining a dried polymer by drying the particulate hydrogel obtained in the polymerization step and/or the gel-grinding step to have a desired resin solids content. The resin solids content of the dried polymer is determined from drying loss (a change in mass after heating 1 g of the water-absorbent resin at 180°C for three hours) and is preferably 80 wt.% or more, more preferably from 85 to 99 wt.%, further preferably from 90 wt.% to 98 wt.%, and particularly preferably from 92 wt.% to 97 wt.%.

A method for drying the particulate hydrogel is not particularly limited, and examples thereof include thermal drying, hot air drying, drying under reduced pressure, fluidized bed drying, infrared drying, microwave drying, drum dryer drying, drying by azeotropic dehydration with a hydrophobic organic solvent, and high humidity drying by use of high temperature water vapor. Among these, from the viewpoint of drying efficiency, hot air drying is preferable, and band drying in which hot air drying is performed on a ventilation belt is more preferable.

The drying temperature (temperature of hot air) in the hot air drying is preferably from 120°C to 250°C, and more preferably from 150 to 200°C, from the viewpoint of the color tone and drying efficiency of the water-absorbent resin. Note that the drying conditions other than the drying temperature, such as the air velocity of hot air and drying time, can be set as appropriate in accordance with the moisture content and total weight of the particulate hydrogel to be dried and the intended resin solids content. When band drying is performed, various conditions disclosed in WO 2006/100300 A, WO 2011/025012 A, WO 2011/025013 A, WO 2011/111657 A, and the like, are applied as appropriate.

(3-5) Pulverizing Step and Classification Step

This step is a step of pulverizing the dried polymer obtained in the drying step (pulverizing step) and adjusting the particle size to a predetermined range (classification step) to obtain a water-absorbent resin powder (a powdery water-absorbent resin before subjected to surface-crosslinking is referred to as "water-absorbent resin powder").

Examples of an apparatus used in the pulverizing step in an embodiment of the present invention include high-speed rotary grinders such as a roll mill, a hammer mill, a screw mill, and a pin mill, a vibration mill, a knuckle-type grinder, and a cylindrical mixer. These are used in combination, as necessary.

In addition, the particle size adjustment method in the classification step in an embodiment of the present invention is not particularly limited, and examples thereof include sieve classification using a JIS standard sieve (JIS Z8801-1 (2000)), airflow classification, or the like. Note that the particle size of the water-absorbent resin is not limited to being adjusted during the pulverizing step and/or the classification step, and may alternatively adjusted as appropriate during the polymerization step (particularly, reverse phase suspension polymerization or spray droplet polymerization) or other steps (for example, a granulation step and a fine powder recovery step).

The weight-average particle size (D50) of the water-absorbent resin powder obtained in the above-described step (the water-absorbent resin powder before the surface-crosslinking step, which is a so-called base polymer) is preferably from 200 to 600 $\mu$m, more preferably from 200 to 550 $\mu$m, further preferably from 250 to 500 $\mu$m, particularly preferably from 300 to 500 $\mu$m. Note that the proportion of the particles having a particle size of less than 150 $\mu$m in the water-absorbent resin powder is preferably 10 wt.% or less, more preferably 5 wt.% or less, further preferably 3 wt.% or less, particularly preferably 1 wt.% or less. The proportion of the particles having a particle size of 850 $\mu$m or more in the water-absorbent resin powder is preferably 5 wt.% or less, more preferably 3 wt.% or less, and further preferably 1 wt.% or less. In either case, the smaller the lower limit of the proportion of these particles, the more preferable it is, and the lower limit is desirably 0 wt.% and may be about 0.1 wt.%. Further, the logarithmic standard deviation ($\sigma\zeta$) of the particle size distribution is preferably from 0.20 to 0.50, more preferably from 0.25 to 0.40, and further preferably from 0.27 to 0.35. When the logarithmic standard deviation ($\sigma\zeta$) of the particle size distribution is excessively small, the range of particle sizes that can be used becomes narrow, the efficiency of the pulverizing and classification steps is significantly reduced, and the productivity of the water-absorbent resin powder is reduced. Therefore, the water-absorbent resin powder in an embodiment of the present invention preferably satisfies the above-described particle size distribution. Note that the particle size is measured using a standard sieve in accordance with the measurement method disclosed in US 7638570 or EDANA ERT420.2-02.

The above-described particle size is applied not only to the water-absorbent resin after surface-crosslinking (hereinafter, may be referred to as "water-absorbent resin particles" for convenience) but also to the particulate water-absorbing agent as a final product. Therefore, the water-absorbent resin particles are preferably subjected to a surface-crosslinking treatment (surface-crosslinking step) so as to maintain the particle size within the above-described range, and more preferably subjected to particle size adjustment by providing a sizing step after the surface-crosslinking step.

(3-6) Surface-Crosslinking Step

This step is a step of further providing a portion with a higher crosslinking density on the surface layer (a portion several tens of micrometers from the surface of the water-absorbent resin powder) of the water-absorbent resin powder obtained through the above-described steps, and it composed of a mixing step, a heat treatment step, and a cooling step (optional).

In the surface-crosslinking step, a surface-crosslinked water-absorbent resin (water-absorbent resin particles)is obtained by radical crosslinking, surface polymerization, crosslinking reaction with a surface-crosslinking agent, or the like, on the surface of the water-absorbent resin powder.

Surface-crosslinking agent

The surface-crosslinking agent used in an embodiment of the present invention is not particularly limited, and examples thereof include an organic or inorganic surface-crosslinking agent. Among these, an organic surface-crosslinking agent that reacts with a carboxyl group is preferable from the viewpoint of the physical properties of the water-absorbent resin and the handleability of the surface-crosslinking agent. Examples thereof include one or more surface-crosslinking agents disclosed in US 7183456. More specific examples include: a polyhydric alcohol compound; an epoxy compound; a haloepoxy compound; a polyamine compound or a condensate thereof with the haloepoxy compound; an oxazoline compound; an oxazolidinone compound; a polyvalent metal salt; an alkylene carbonate compound; and a cyclic urea compound.

Specific examples of the organic surface-crosslinking agent include: polyhydricalcohol compounds such as (di, tri, tetra, poly)ethylene glycol, (di, poly)propylene glycol, 1,3-propanediol, 2,2,4,-trimethyl-1, 3-pentanediol, (poly)glycerin, 2-butene-1,4-diol, 1,4-butanediol, 1,3-butanediol, 1,5-pentanediol, 1,6-hexanediol, trimethylolpropane, di- or triethanolamine, pentaerythritol, and sorbitol; epoxy compounds such as (poly)ethylene glycol diglycidyl ether, (di, poly)glycerol polyglycidyl ether, and glycidol; oxazoline compounds such as 2-oxazolidone, N-hydroxyethyl-2-oxazolidone, and 1,2-ethylenebisoxazoline; alkylene carbonate compounds such as 1,3-dioxolan-2-one (ethylene carbonate), 4-methyl-1, 3-dioxolan-2-one, 4,5-dimethyl-1,3-dioxolan-2-one, 4,4-dimethyl-1, 3-dioxolan-2-one, 4-ethyl-1,3-dioxolan-2-one, 4-hydroxymethyl-1, 3-dioxolan-2-one, 1,3-dioxan-2-one, 4-methyl-1,3-dioxan-2-one, 4, 6-dimethyl-1,3-dioxan-2-one, and 1,3-dioxopan-2-one; haloepoxy compounds such as epichlorohydrin, epibromohydrin, and $\alpha$-methylepichlorohydrin, and polyvalent amine adducts thereof (for example, Kymene available from Hercules Incorporated; registered trademark); silane coupling agents such as $\gamma$-glycidoxypropyltrimethoxysilane and $\gamma$-aminopropyltriethoxysilane; oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, 3-butyl-3-oxetaneethanol, 3-chloromethyl-3-methyloxetane, 3-chloromethyl-3-ethyloxetane, and polyvalent oxetane compounds; and cyclic urea compounds such as 2-imidazolidinone.

The polyhydric alcohol is preferably a polyhydric alcohol having 2 to 8 carbon atoms, more preferably a polyhydric alcohol having 3 to 6 carbon atoms, and further preferably a polyhydric alcohol having 3 or 4 carbon atoms. Further, as the polyhydric alcohol, a diol is particularly preferable. Examples of the diol include ethylene glycol, propylene glycol, 1,3-propanediol, and 1,4-butanediol. Among these, as the polyhydric alcohol, one or more polyhydric alcohols selected from propylene glycol(1,2-propanediol), 1,3-propanediol, and 1,4-butanediol are preferable.

In addition, the epoxy compound is preferably a polyglycidyl compound, and ethylene glycol diglycidyl ether is preferably used.

In addition to the organic surface-crosslinking agent, a polyvalent cationic polymer such as a polyamine polymer and/or a water-soluble polyvalent metal cation-containing compound may be used in combination as an ionic surface-crosslinking agent from the viewpoint of more effectively performing surface-crosslinking. The water-soluble polyvalent metal cation-containing compound includes the ones described above. These polyvalent cationic polymers and/or water-soluble polyvalent metal cation-containing compounds may be added to a surface-crosslinking agent solution to be described later and mixed with the water-absorbent resin powder, or may be added to the water-absorbent resin powder and mixed with the water-absorbent resin powder separately from the surface-crosslinking agent solution.

The amount of the surface-crosslinking agent used (the total amount used when a plurality of agents are used) is preferably from 0.01 to 10 parts by weight, more preferably from 0.01 to 5 parts by weight, per 100 parts by weight of the water-absorbent resin powder. In addition, the surface-crosslinking agent is preferably added as an aqueous solution, and in this case, the amount of water used is preferably from 0.1 to 20 parts by weight, more preferably from 0.5 to 10 parts by weight, per 100 parts by weight of the water-absorbent resin powder. Further, when a hydrophilic organic solvent is used as necessary, the amount of the organic solvent used is preferably 10 parts by weight or less, more preferably 5 parts by weight or less, per 100 parts by weight of the water-absorbent resin powder.

Mixing Step

This step is a step of mixing the water-absorbent resin powder and the surface-crosslinking agent. The method for mixing the surface-crosslinking agent is not particularly limited, and examples thereof include a method for preparing a surface-crosslinking agent solution previously, and preferably spraying or dropping, more preferably spraying the solution to a water-absorbent resin powder to mix them.

An apparatus for mixing is not particularly limited, and a high-speed stirring type mixer is preferable, and a high-speed stirring type continuous mixer is more preferable.

Heat Treatment Step

This step is a step of causing a crosslinking reaction on the surface of the water-absorbent resin powder by applying heat to the mixture obtained in the mixing step.

An apparatus for performing the crosslinking reaction is not particularly limited, and a paddle dryer is preferable. The reaction temperature in the crosslinking reaction is appropriately set depending on the type of the surface-crosslinking agent used, and is preferably from 50 to 300°C, more preferably from 80 to 200°C.

Cooling Step

This cooling step is an optional step provided, as necessary, after the heat treatment step.

The apparatus for performing the cooling is not particularly limited and is preferably an apparatus having the same specification as the apparatus used in the heat treatment step, and more preferably a paddle dryer. This is because changing the heat medium to a refrigerant allows the apparatus to be used as a cooling apparatus. The water-absorbent resin particles obtained in the heat treatment step are forcibly cooled, as necessary, to preferably from 40 to 80°C, more preferably from 50 to 70°C, in the cooling step.

(3-7) Additive Adding Step

This step is a step of adding additives such as a polyvalent metal salt, a cationic polymer, a chelating agent, an inorganic reducing agent, a hydroxycarboxylic acid compound, water-insoluble inorganic particles, inorganic colloidal particles, a water-soluble polyvalent metal cation-containing compound, a surfactant, and a non-polymer water-soluble compound to the water-absorbent resin particles obtained in the surface-crosslinking step. The additive can be mixed with the water-absorbent resin powder simultaneously with the surface-crosslinking agent (aqueous solution).

Polyvalent Metal Salt and/or Cationic Polymer

From the viewpoint of improving the water absorption speed, liquid permeability, hygroscopic fluidity, and the like, of the obtained particulate water-absorbing agent, a polyvalent metal salt and/or a cationic polymer may be added to the water-absorbent resin particles obtained in the surface-crosslinking step.

As the polyvalent metal salt and/or the cationic polymer, specifically, the compounds and their amounts used as disclosed in "[7] Polyvalent Metal Salt and/or Cationic Polymer" of WO 2011/040530 are applied to an embodiment of the present invention.

Chelating Agent

From the viewpoint of the color tone (suppression of coloring), the suppression of deterioration, and the like, of the obtained particulate water-absorbing agent, a chelating agent may be added to the water-absorbent resin particles obtained in the surface-crosslinking step.

As the chelating agent, for example, the compounds and the amounts thereof used as in "[2] Chelating Agent" of WO 2011/040530 are applied to an embodiment of the present invention.

Inorganic Reducing Agent

From the viewpoint of the color tone (suppression of coloring), the suppression of deterioration, reduction of residual monomers, and the like of the obtained particulate water-absorbing agent, an inorganic reducing agent may be added to the water-absorbent resin particles obtained in the surface-crosslinking step.

As the inorganic reducing agent, for example, the compounds and the amounts thereof used as in "[3] Inorganic Reducing Agent" of WO 2011/040530 are applied to an embodiment of the present invention.

$\alpha$-Hydroxycarboxylic Acid Compound

From the viewpoint of the color tone (suppression of coloring) of the obtained particulate water-absorbing agent, an $\alpha$-hydroxycarboxylic acid may be added to the water-absorbent resin particles obtained in the surface-crosslinking step. The "$\alpha$-hydroxycarboxylic acid compound" refers to a carboxylic acid having a hydroxyl group in the molecule or a salt thereof, and is a hydroxycarboxylic acid having a hydroxyl group at the $\alpha$-position.

As the $\alpha$-hydroxycarboxylic acid compound, for example, the compounds and the amounts thereof used as in "[6] $\alpha$-Hydroxycarboxylic Acid Compound" of WO 2011/040530 are applied to an embodiment of the present invention, and malic acid (salt) and lactic acid (salt) are particularly preferable.

Water-Insoluble Inorganic Particles

From the viewpoint of improving the fluidity of the particulate water-absorbing agent, water-insoluble inorganic particles may be added to the water-absorbent resin particles obtained in the surface-crosslinking step. Examples of the water-insoluble inorganic particles to be added to the water-absorbent resin particles obtained in the surface-crosslinking step include the water-insoluble inorganic particles described in the section (2-6).

Inorganic Colloidal Particles

From the viewpoint of improving the physical properties of the particulate water-absorbing agent to be obtained, inorganic colloidal particles may be added to the water-absorbent resin particles obtained in the surface-crosslinking step. Examples of the inorganic colloidal particles added to the water-absorbent resin particles obtained in the surface-crosslinking step include the inorganic colloidal particles described in the section (2-6).

Water-Soluble Polyvalent Metal Cation-Containing Compound

From the viewpoint of improving the performance of the particulate water-absorbing agent to be obtained, a water-soluble polyvalent metal cation-containing compound may be added to the water-absorbent resin particles obtained in the surface-crosslinking step. Examples of the water-soluble polyvalent metal cation-containing compound include the water-soluble polyvalent metal cation-containing compounds described in the section (2-6).

Surfactant

From the viewpoint of improving the physical properties (for example, water absorption speed) of the resulting water-absorbent resin, a surfactant may be added to the water-absorbent resin particles obtained in the surface-crosslinking step.

Examples of the surfactant include the surfactants disclosed in WO 97/017397 or US 6107358, that is, nonionic surfactants, anionic surfactants, cationic surfactants, and amphoteric surfactants.

Non-Polymer Water-Soluble Compound

From the viewpoint of reducing dust of the water-absorbent resin, a non-polymer water-soluble compound may be added to the water-absorbent resin particles obtained in the surface-crosslinking step. As the non-polymer water-soluble compound, for example, the compounds and the amounts thereof as disclosed in "Non-Polymer Water-Soluble Compound" of WO 2014/034667 are applied to an embodiment of the present invention.

In an embodiment of the present invention, an additive other than the above-described additives can be added to the water-absorbent resin particles obtained in the surface-crosslinking step in order to add various functions to the water-absorbent resin. Examples of the additive include a compound having a phosphorus atom, an oxidizing agent, an organic reducing agent, an organic powder such as a metal soap, a deodorant, an antibacterial agent, pulp, and thermoplastic fiber.

The amount of the additive used (added) is not particularly limited, and it is appropriately determined depending on the application. The amount used (added) is preferably 3 parts by weight or less, more preferably 1 part by weight or less, per 100 parts by weight of the water-absorbent resin. In addition, the additive can be added in a step different from the above-described step.

(3-8) Other Steps

In an embodiment of the present invention, in addition to the above-described steps, a granulation step, a sizing step, a fine powder removal step, a fine powder recycling step, and the like can be provided, as necessary. In addition, one or more steps such as a transportation step, a storage step, a packaging step, and a preservation step are further included. The "sizing step" includes a fine powder removal step after the surface-crosslinking step, and a step of classifying and pulverizing the water-absorbent resin when the water-absorbent resin is aggregated and exceeds a desired size. The "fine powder recycling step" includes a step of adding not only the fine powder as it is as in the present invention but also the fine powder in the form of a large hydrogel to any step in the production process of the water-absorbent resin.

(3-9) Method for Controlling SRC (NP) and SRC (4.83 kPa)

Employing the following control method in the steps presented in the above-described production steps (3-1) to (3-8) makes it possible to control SRC (NP) and SRC (4.83 kPa) of the particulate water-absorbing agent to be produced within a preferable range. This makes it possible to suitably produce the particulate water-absorbing agent of the present invention.

Examples of the control method include performing gel-grinding described in the section of "(3-3) Gel-Grinding Step" under specific conditions. Specific examples thereof include performing the gel-grinding by controlling the gel-grinding energy (GGE) to preferably from 15 to 40 J/g, more preferably from 20 to 40 J/g, further preferably from 20 to 35 J/g, and particularly preferably from 25 to 35 J/g. Performing the gel-grinding in this manner allows the gel-grinding so as to apply an appropriate shearing force and an appropriate compressive force to the hydrogel. As a result, the particle shape and particle size distribution of the hydrogel can be controlled, and the SRC (NP) and the SRC (4.83 kPa) of the particulate water-absorbing agent to be produced can be controlled to be within the above-described preferable ranges.

Herein, the "gel-grinding energy" in an embodiment of the present invention refers to unit energy required by a gel-grinding apparatus when a hydrogel is ground, that is, mechanical energy per unit mass of the hydrogel. The energy for heating and cooling the jacket and the energy of the water and steam to be charged are not included in the gel-grinding energy. The "gel-grinding energy" is abbreviated as "GGE" from "Gel-Grinding Energy" in English.

GGE is calculated by the following Formula (6) when the gel-grinding apparatus is driven by three phase AC power:

$$\text{GGE [J/g]} = \{\sqrt{3} \times \text{voltage} \times \text{current} \times \text{power factor} \times \text{motor- efficiency}\}/\{\text{mass of hydrogel charged into gel-grinding apparatus per second}\} \qquad \text{Formula (6)}$$

The "power factor" and the "motor efficiency" are values that vary depending on the operating conditions of the gel-grinding apparatus and are specific to the gel-grinding apparatus, and the values thereof are 0 or more and 1 or less. These values can be known by making inquiries to the device manufacturer or the like. In addition, when the gel-grinding apparatus is driven by single-phase AC power, the GGE can be calculated by changing "$\sqrt{3}$" in the above-described Formulas (6) to "1". The unit of voltage is [V], the unit of current is [A], and the unit of mass of the hydrogel is [g/s].

The "power factor" and the "motor efficiency" in the GGE are values during gel-grinding. The power factor and the motor efficiency during the idling operation are approximately defined as in Formula (6) because the current value during the idling operation is small. The "mass of the hydrogel to be charged into the gel-grinding apparatus per second" [g/s] in Formula (6) refers to a value converted into [g/s] when the hydrogel is continuously supplied by a quantitative feeder, for example. However, as described later, the hydrogel may include a recycled granulated gel.

In addition, in the present invention, the mechanical energy applied to the hydrogel is important. Therefore, it is preferable to calculate the gel-grinding energy using a current value obtained by subtracting a current value when the gel-grinding apparatus is idling from a current value during gel-grinding as a value of "current" in Formula (6). In particular, when gel-grinding is performed with a plurality of apparatuses, the total of the current values during idling increases, and therefore, a method for calculating the GGE using a value obtained by subtracting the current value during idling from the current value during gel-grinding as the value of "current" in the above-described Formula (6) is suitable.

In the present invention, the CRC of the water-absorbent resin particles obtained by pulverizing the hydrogel by applying a high GGE of 15 J/g or more in the gel-grinding step and then performing the drying step and the pulverizing and classification step described above after the gel-grinding step is preferably from 50 to 70 g/g, more preferably from 50 to 65 g/g, further preferably from 51 to 63 g/g, and particularly preferably from 52 to 62 g/g. However, it is significantly difficult to control the CRC of the water-absorbent resin particles, which are obtained by pulverizing the hydrogel by applying a high GGE of 15 J/g or more and then performing the drying step and the pulverizing and classification step after the gel-grinding step, to the preferable range. The reason is shown below.

In order to increase the CRC of the water-absorbent resin particles, the hydrogel obtained in the polymerization step needs to be a low-crosslinked gel having a relatively low crosslinking density. Such a low-crosslinked gel is difficult to be ground by applying a high GGE of 15 J/g or more. Therefore, it is difficult to apply an appropriate shear force and compression force to the low-crosslinked gel in order to control the SRC (NP) and the SRC (4.83 kPa) to the above-described preferable ranges.

The present inventors have made intensive studies on these issues and have made the dischargeability of the hydrogel from the grinder lower than before. Examples of the method for reducing the dischargeability of the hydrogel include a method for installing a weir at the outlet of the grinder, a method for reducing the size of the discharge port of the grinder, and a method for adjusting a perforated plate installed at the outlet of the grinder. Accordingly, when the hydrogel supplied to the grinder is a low-crosslinked gel, energy can be easily applied to the low-crosslinked gel, and an appropriate shear force and compression force can be applied to the low-crosslinked gel. As a result, the SRC (NP) and SRC (4.83 kPa) improved.

When gel-grinding is performed by a plurality of apparatuses, such as the use of a screw extruder after kneader polymerization or the use of a plurality of screw extruders, the total of the energy consumed by each apparatus is defined as the gel-grinding energy (GGE).

In addition, examples of the control method include, in addition to the method for performing gel-grinding under the specific conditions described above, a method for forming the water-absorbent resin particles into a foamed shape, and a method for performing polymerization by reducing the monomer concentration in the aqueous monomer solution in the polymerization step. Forming the water-absorbent resin particles into the foamed shape allows the surface area of the water-absorbent resin particles to increase, and the liquid retention capacity of the particulate water-absorbing agent to be improved. Note that employing a method for polymerizing the monomers while lowering the monomer concentration allows the performance of the particulate water-absorbing agent to be improved. As a result, the SRC (NP) and the SRC (4. 83 kPa) of the particulate water-absorbing agent to be produced can be controlled to be within the above-described preferable ranges.

[4] Use of Particulate Water-Absorbing Agent

The particulate water-absorbing agent of the present invention is used for applications for water absorption and is widely used as an absorbent body. In addition, the particulate water-absorbing agent of the present invention can be used for an absorbent article containing the absorbent body. In particular, the particulate water-absorbing agent of the present invention is suitably used as a sanitary article for absorbing body fluids such as urine and blood and suitably used as a sanitary article for a person among absorbent articles because re-wetting under pressure is reduced.

That is, a preferable embodiment of the present invention is an absorbent body including the particulate water-absorbing agent of the above-described form. In addition, another preferable embodiment of the present invention is a sanitary article containing the absorbent body. The absorbent body includes the particulate water-absorbing agent of the present invention, and thus a sanitary product containing the absorbent body can further reduce re-wetting of absorbed liquid during actual use.

Examples of the absorbent body include an absorbent material molded by using the particulate water-absorbing agent and a fiber base material (for example, hydrophilic fiber) as main components. The content (core concentration) of the particulate water-absorbing agent per the total weight of the particulate water-absorbing agent and the hydrophilic fiber in the absorbent body is preferably from 10 to 100 wt.%, more preferably from 15 to 90 wt.%, particularly preferably from 20 to 80 wt.%, and most preferably from 25 to 80 wt.%. As the core concentration in the absorbent body is higher, the water absorption performance of the absorbent body and the sanitary product including the absorbent body is more affected by the water absorption performance of the particulate water-absorbing agent included in the absorbent body and the sanitary product. The particulate water-absorbing agent contained in the absorbent body and the sanitary product is a particulate water-absorbing agent used in the production of the absorbent body and the sanitary product. Such an absorbent body is molded by blending or sandwiching a fiber base material such as hydrophilic fibers and the particulate water-absorbing agent. Examples of the fiber base material to be used include hydrophilic fibers such as ground wood pulp, cotton linters, crosslinked cellulose fibers, rayon, cotton, wool, acetate, and vinylon. These fiber base materials are preferably those formed by airlaid.

In addition, the absorbent body may be a (pulp-less) water absorbent sheet in which a water-absorbent resin is fixed between two sheets (for example, nonwoven fabrics).

In addition, the above-described absorbent article is an absorbent article containing the absorbent body, a liquid-permeable top sheet, and a liquid-impermeable back sheet. For the above-described absorbent article, an absorbent body (absorbent core) is produced, and the absorbent core is sandwiched between a liquid-permeable top sheet and a liquid-impermeable back sheet. Thereafter, an absorbent article such as a disposable diaper for adults or a sanitary napkin is obtained by providing an elastic member, a diffusion layer, an adhesive tape, or the like, as necessary. In this case, the absorbent core is compression-molded to have a density of, for example, from 0.06 to 0.50 $[g/cm^3]$ and a basis weight from 0.01 to 0.20 $[g/cm^2]$.

An embodiment of the present invention may include inventions described in [1] to [8] set forth below.

[1] A particulate water-absorbing agent, containing, as a main component, a surface-crosslinked polyacrylic acid (salt)-based water-absorbent resin, the particulate water-absorbing agent satisfying the following Formula (A) and the following Formula (B).

$$\text{SRC (NP)} > 50.0 \text{ g/g (A)}$$

$$\text{SRC (4.83 kPa)} > 41.5 \text{ g/g (B)}$$

Herein, the SRC (NP) is measured by a method including the following steps (a) to (c).

(a) Obtaining a swollen gel by bringing the particulate water-absorbing agent $(W_0)$ [g] into contact with a 0.9% aqueous sodium chloride solution (hereinafter referred to as a "test solution") for 10 minutes with no load applied.
(b) Separating the swollen gel obtained in the step (a) and the test solution.
(c) Measuring a weight $(W_1)$ [g] of the swollen gel separated in the step (b) and calculating SRC (NP) of the particulate water-absorbing agent based on the following Formula (1):

$$\text{SRC (NP) } [\text{g/g}] = W_1/W_0 \text{ (1)}$$

The SRC (4.83 kPa) is measured by the method including the steps (a) to (c) except that, instead of the step (c), steps (c') to (e') described below are performed.

(c') Applying a load of 4.83 kPa to the swollen gel separated in the step (b) for 1 minute.

(d') Removing an exudate exuded from the swollen gel in the step (c').

(e') Measuring a weight ($W_2$) [g] of the swollen gel obtained in the step (d'), and calculating the SRC (4.83 kPa) based on the following Formula (2):

$$\text{SRC (4.83 kPa) } [\text{g/g}] = W_2/W_0 \text{ (2)}$$

[2] The particulate water-absorbing agent according to [1], wherein a value of SRC (4.83 kPa) in Formula (B) is more than 42 g/g.

[3] The particulate water-absorbing agent according to [1] or [2], wherein a value of SRC (NP) in Formula (A) is more than 54.

[4] The particulate water-absorbing agent according to any one of [1] to [3], wherein a water absorption speed (Vortex method) is 40 seconds or less.

[5] The particulate water-absorbing agent according to any one of [1] to [4], wherein a surface tension (STR) is 66 mN/m or more.

[6] The particulate water-absorbing agent according to any one of [1] to [5], wherein a centrifuge retention capacity (CRC) is 35 g/g or more.

[7] The particulate water-absorbing agent according to [6], wherein a centrifuge retention capacity (CRC) is 40 g/g or more.

[8] The particulate water-absorbing agent according to any one of [1] to [7], further containing one or more selected from the group consisting of inorganic colloidal particles, water-insoluble inorganic particles, and water-soluble polyvalent metal cation-containing compounds.

[9] An absorbent body, including the particulate water-absorbing agent according to any one of [1] to [8].

[10] A sanitary product, including the absorbent body according to [9].

Examples

The present invention will be described in more detail with reference to the following Examples and Comparative Examples. However, the technical scope of the present invention is not limited only to the following Examples. In addition, in the following Examples, unless otherwise specified, the operations were performed under the conditions of room temperature (from 20 to 25°C) and relative humidity of from 45 to 55 %RH.

Unless otherwise noted, for the electric devices (also including the measurement of physical properties of the particulate water-absorbing agent) used in the Examples, Comparative Examples, and Reference Examples, a power supply of 200 V or 100 V was used.

(a) Method for Measuring SRC (NP)

The SRC (NP) measurement was performed using the cylinder and piston as used in the Gel Bed Permeability test disclosed in US 8269060. In the bottom surface of the cylinder, a 400-mesh wire mesh was attached, which did not allow the particulate water-absorbing agent and the swollen gel described later to pass and allowed the test solution and the exudate described later to be sucked or discharged.

First, the total weight of the cylinder and piston used for the measurement of SRC (NP) was measured, and the value was defined as $W_a$ [g]. Subsequently, a sample of the particulate water-absorbing agent to be measured was weighed out to approximately 0.9 g and uniformly scattered on the bottom surface of the cylinder. Herein, the exact weight of the sample is defined as $W_0$ [g]. Subsequently, the cylinder was placed in a large excess of a 0.9% aqueous sodium chloride solution adjusted to a temperature of 23 $\pm$ 1°C as a test solution. Thus, the uniformly scattered sample was brought into contact with the test solution for 10 minutes, and the sample was allowed to absorb the test solution and swell with no load applied, thereby providing a swollen gel.

Then, a piston (approximately 72 g) was placed on the swollen gel in the cylinder, and the cylinder was pulled up from the test solution. The cylinder pulled up from the test solution was placed on a JIS standard sieve having a mesh size of 2000 μm. Herein, when the cylinder was pulled up from the test solution and placed on the JIS standard sieve, the solution remained in the cylinder, which the swollen gel could not retain, that is, the excess water was drained from the bottom surface of the cylinder. That is, the excess water that could not be retained by the swollen gel was removed.

The cylinder was allowed to stand on the JIS standard sieve for 1 minute to remove excess water from the swollen gel particles. That is, after 1 minute from the start of the draining, that is, from the time when the cylinder was placed on the JIS standard sieve, no water droplet dropped from the measuring apparatus for five seconds. Thereafter, the cylinder, the swollen gel and the piston were weighed. In this case, when water droplets were on the bottom surface and/or the side surface of the cylinder, the water droplets were removed by Kimwipes (S-200 available from NIPPON PAPER CRECIA CO., LTD.) or the like, before the measurement of the weight. The weight of the cylinder, the swollen gel, and the piston obtained is defined as $W_b$ [g]. Using the aforementioned $W_0$, $W_a$, and $W_b$, SRC (NP) of the particulate water-absorbing agent constituting the sample was calculated based on the following Formula (1'). In Formula (1') described later, ($W_b$ [g] - $W_a$ [g]) is the weight of the swollen gel after being let stand in the step (b) described above, which is $W_1$ [g].

$$\text{SRC (NP) [g/g]} = (W_b \text{ [g]} - W_a \text{ [g]})/W_0 \text{ [g]} \quad (1')$$

(b) Method for Measuring SRC (4.83 kPa)

The SRC (4.83 kPa) measurement was performed using the cylinder, piston and weight as used in the Gel Bed Permeability test disclosed in US 8269060. In the bottom surface of the cylinder, a 400-mesh wire mesh was attached, which did not allow the particulate water-absorbing agent and the swollen gel described later to pass and allowed the test solution and the exudate described later to be sucked or discharged.

First, the total weight of the cylinder, piston, and weight used for the measurement of SRC (4.83 kPa) was measured, and the value was defined as $W_c$ [g]. Subsequently, a sample of the particulate water-absorbing agent to be measured was weighed out to approximately 0.9 g and uniformly scattered on the bottom surface of the cylinder. Herein, the exact weight of the sample is defined as $W_0$ [g]. Subsequently, the cylinder was placed in a large excess of a 0.9% aqueous sodium chloride solution adjusted to a temperature of $23 \pm 1$°C as a test solution. Thus, the uniformly scattered sample was brought into contact with the test solution for 10 minutes, and the sample was allowed to absorb the test solution and swell with no load applied, thereby providing a swollen gel.

Then, a piston (approximately 72 g) was placed on the swollen gel in the cylinder, and the cylinder was pulled up from the test solution. The cylinder pulled up from the test solution was placed on a JIS standard sieve having a mesh size of 2000 μm. Herein, when the cylinder was pulled up from the test solution and placed on the JIS standard sieve, the solution remained in the cylinder, which the swollen gel could not retain, that is, the excess water, was drained from the bottom surface of the cylinder. That is, the excess water that could not be retained by the swollen gel was removed.

The cylinder was allowed to stand on the JIS standard sieve for 1 minute to remove excess water from the swollen gel particles. That is, after 1 minute from the start of the draining, that is, from the time when the cylinder was placed on the JIS standard sieve, no water droplet dropped from the measuring apparatus for five seconds. Thereafter, the weight (approximately 1316 g) was additionally placed on the piston. In this case, the load applied to the swollen gel was 4.83 kPa. The above-described operation allowed the solution, which was retained in between the swollen gels before pressure was applied, to be exuded. A part of the solution that had been exuded, that is, the exudate leaked to the lower part of the swollen gel, that is, onto the bottom surface of the cylinder, was drained from the bottom surface. On the other hand, the rest of the exudate was accumulated on the upper side of the swollen gel, that is, the upper portion of the piston. One minute after the weight was placed on the piston, the exudation of the exudate stopped and a steady state was reached, and therefore the rest of the exudate accumulated on the upper portion of the piston was rapidly removed using a dropper. After the exudate exuded from the swollen gel was removed as described above, the weights of the cylinder, the piston, the weight, and the swollen gel were measured. In this case, when water droplets were on the bottom surface and/or the side surface of the cylinder, the water droplets were removed by Kimwipes (S-200 available from NIPPON PAPER CRECIA CO., LTD.) or the like, before the measurement of the weight. The weight of the cylinder, the piston, the weight, and the swollen gel thus obtained is defined as $W_d$ [g]. Using the above-described $W_0$, $W_c$, and $W_d$, SRC (4.83 kPa) of the particulate water-absorbing agent constituting the sample was calculated based on the following Formula (2'). In Formula (2') described later, ($W_d$ [g] - $W_c$ [g]) is the weight of the swollen gel after removal of the exudate in the step (d') described above: $W_2$ [g].

$$\text{SRC (4.83 kPa) [g/g]} = (W_d \text{ [g]} - W_c \text{ [g]})/W_0 \text{ [g]} \quad (2')$$

(c) Method for Measuring CRC

The centrifuge retention capacity (absorption capacity without pressure, CRC) of the particulate water-absorbing agent or the water-absorbent resin was measured in accordance with the EDANA method (ERT441.2-02).

(d) Particle Size Distribution (Particle Size Distribution, Weight-Average Particle Size (D50), and Logarithmic Standard Deviation ($\sigma\zeta$) of Particle Size Distribution)

The particle size distributions (particle size distributions, weight-average particle sizes (D50), and logarithmic standard deviations ($\sigma\zeta$) of particle size distributions) of the particulate water-absorbing agent or the water-absorbent resin are measured in accordance with "(3) Mass-Average Particle Diameters (D50) and Logarithmic Standard Deviations ($\sigma\zeta$) of Particle Diameter Distribution" disclosed in columns 27 and 28 of US 7638570.

(e) Method for Measuring Water Absorption Speed (Vortex Method)

0.02 parts by weight of Food Blue No. 1, a food additive, was added to 1000 parts by weight of a 0.90 wt.% aqueous sodium chloride solution prepared previously, and the liquid temperature was adjusted to 30°C. 50 ml of a 0.90 wt.% aqueous sodium chloride solution colored blue was weighed out in a 100 ml beaker, and 2.00 g of the particulate water-absorbing agent was added while stirring at 600 rpm using a cylindrical stirrer having a length of 40 mm and a thickness of 8 mm, and the water absorption time (seconds) was measured. The end point was determined according to the standard described in JIS K7224-1996 "Explanation of the Absorption Rate Test Method for Superabsorbent Polymers", the time from when the water-absorbing agent absorbed saline until the test solution covered the stirrer tip was measured as the water absorption speed (seconds).

(f) Method for Measuring Surface Tension (STR)

50 ml of saline adjusted to 20°C and a sufficiently washed rotor made of fluorine resin having a 25 mm length were put into a sufficiently washed beaker of 100 ml, and first, the surface tension of the saline was measured using a surface tensiometer (K11 automatic surface tensiometer available from KRUSS GmbH). In this measurement, it was confirmed that the value of the surface tension was in a range from 71 to 75 [mN/m].
Subsequently, 0.5 g of the particulate water-absorbing agent was put into a beaker containing the saline adjusted to 20°C, the surface tension of which was measured, and the mixture was stirred for 4 minutes under the condition of 500 rpm. After 4 minutes, the stirring was stopped, and after the particulate water-absorbing agent containing water was precipitated, the surface tension of the supernatant was measured by performing the same operation as the operation of measuring the surface tension of the only saline. In the present examples, a plate method using a platinum plate was adopted, and the plate was used after being sufficiently washed with deionized water and heated and washed with a gas burner before each measurement.

(g) Method for Measuring Absorption Amount by Absorbent Body

Preparation of Absorbent Body A

An 80 mm × 160 mm absorbent cotton (cut cotton 8 cm × 16 cm available from KAWAMOTO CORPORATION) was uniformly torn in the plane direction, and two sheets of 2.8 g absorbent cotton were prepared. Then, a water absorbent paper was cut into 200 mm × 200 mm, and a frame of 8 cm × 16 cm was disposed near the center of the cut-out water absorbent paper. One of the absorbent cotton sheets having a weight of 2.8 g was laid in the frame, and the upper surface of the absorbent cotton sheet was leveled with an acrylic plate or the like. 3 g of the particulate water-absorbing agent was uniformly scattered on the upper surface, and the other absorbent cotton sheet having a weight of 2.8 g was placed thereon to form a sand structure. Further, the whole inside of the frame was held for 1 minute in a state where the load of 10 kg was applied, and an absorbent body was molded. Thereafter, the load and the frame were removed, and both ends of the water absorbent paper were folded back along the longitudinal direction of the absorbent body so as to wrap the absorbent body. The obtained absorbent body was placed in a nonwoven fabric bag (10 cm × 22 cm) prepared using Heatron paper, and the periphery was heat-sealed to prepare an absorbent body A.

Measurement of Absorption Amount of Absorbent Body

A 0.9% aqueous sodium chloride solution was placed in a deep vat such that the liquid depth was equal to or higher than 5 cm, and the temperature of the liquid in the vat was adjusted to 25°C. The absorbent body A was immersed in the vat and left to stand for 10 minutes in a state where no load was applied, thereby swelling the absorbent body A. After swelling, the swollen absorbent body A was taken out from the vat while maintaining a horizontal state with respect to the liquid surface in the vat, and placed on a JIS standard sieve having a diameter of 45 cm and a mesh size of 2000 $\mu$m while maintaining the horizontal state. Subsequently, an acrylic plate was placed on the upper surface of the absorbent body A, at a portion having an area of 8 cm × 16 cm where the particulate water-absorbing agent was present in the absorbent body A, a weight with total weight with the acrylic plate being 6405 g was placed thereon, and liquid draining was performed for 1 minute. The pressure applied by the acrylic plate and the weight to the portion of the absorbent body A on which the acrylic plate was placed was 50 g/cm$^2$. The weight of the absorbent body A after the liquid draining was measured, and the difference from the weight of the absorbent body A before immersion measured previously was calculated as the "absorption amount of the absorbent body". The calculated values are shown in Table 2.

(h) Method for Measuring Amount of Re-Wetting from Absorbent Body

Method for Preparing Absorbent Body B

A vinyl tape (vinyl tape 21-100TM available from Nitto Denko Corporation) was cut into a 100 mm × 200 mm, the adhesive surface was placed facing upward, and a frame of 80 mm × 160 mm was placed thereon. An 80 mm × 160 mm absorbent cotton (cut cotton 8 cm × 16 cm available from KAWAMOTO CORPORATION) was uniformly torn in the plane direction to prepare a 2.8 g absorbent cotton sheet, which was then placed in the frame. Further, on the absorbent cotton sheet in the frame, 1.5 g of the particulate water-absorbing agent was scattered uniformly. The frame was removed, an air-through nonwoven fabric separately cut out into a size of 100 mm × 200 mm was placed on the particulate water-absorbing agent, and the outer peripheral portion of the air-through nonwoven fabric was bonded to a vinyl tape to provide an absorbent body B.

Measurement of Re-Wetting Amount from Absorbent Body

70 mL of a 0.9% aqueous sodium chloride solution was charged into the central portion of the absorbent body B. The aqueous sodium chloride solution was charged using a liquid charging apparatus including an 80 mm × 160 mm flat sheet having a circular hole with a 20 mm inside diameter at the center thereof and a cylinder with a 20 mm inside diameter and an 80 mm length at the center thereof. The inside of the cylinder and the circular hole were in communication with each other. The liquid charging apparatus was placed on the upper surface of the absorbent body B and at a portion of the 8 cm × 16 cm portion where the particulate water-absorbing agent was present in the absorbent body B, that is, on the central portion, and the aqueous sodium chloride solution was charged into the central portion of the absorbent body B through the cylinder and the circular hole by using a funnel having a flow rate of 7 mL/sec. Ten minutes after the start of charging the aqueous sodium chloride solution, 30 sheets of 80 mm × 80 mm filter paper, the weight of which had been measured previously, were stacked and placed in the central portion, and the entire filter paper was pressurized with a 3200 g weight for 1 minute. In this case, the liquid leaked from the absorbent body B, and the liquid was absorbed by the filter paper. As the above-described filter paper, a filter paper obtained by cutting 100 mm × 100 mm Model No. 2 available from ADVANTEC into 80 mm × 80 mm was used. The difference between the weight of the filter paper before use and the weight of the filter paper after absorbing the liquid was calculated as the "re-wetting amount from the absorbent body". The calculated values are shown in Table 2.

Production Example 1

An aqueous monomer solution (1) including 300 parts by weight of acrylic acid, 123.6 parts by weight of a 48 wt.% aqueous sodium hydroxide solution, 0.44 parts by weight of polyethylene glycol diacrylate (average n number: 9) (hereinafter referred to as "PEGDA-9"), 1.18 parts by weight of a 31 wt.% aqueous ethylenediaminetetramethylenephosphonic acid solution, and 331.8 parts by weight of deionized water was prepared.

Then, the aqueous monomer solution (1) adjusted to 41°C was continuously supplied to a continuous polymerization apparatus having a planar polymerization belt provided with weirs at both ends of the belt by a metering pump, and polymerization was performed. Before being supplied to the continuous polymerization apparatus, 127.0 parts by weight of a 48 wt.% aqueous sodium hydroxide solution was continuously mixed with the aqueous monomer solution (1) by a line mixer. After the mixing, the liquid temperature of the mixture of the aqueous monomer solution (1) and the 48 wt.% aqueous sodium hydroxide solution was raised to 80°C by the heat of neutralization.

The obtained aqueous monomer solution (1) was continuously mixed with 10.6 parts by weight of a 5 wt.% aqueous sodium persulfate solution by a line mixer to provide a mixed solution. The mixed solution was continuously supplied to the continuous polymerization apparatus such that the liquid depth on the polymerization belt became 10 mm. A polymerization reaction occurred on the polymerization belt, and a belt-shaped hydrogel (1) was obtained. The polymerization time of the polymerization was 3 minutes. The obtained belt-shaped hydrogel (1) was continuously cut at equal intervals in the widthwise direction with respect to the traveling direction of the polymerization belt such that the cut length was 300 mm, thereby providing a hydrogel (1).

The hydrogel (1) was supplied to a screw extruder, and gel-grinding of the hydrogel (1) was performed. The screw extruder was a meat chopper provided with a perforated plate having a 100 mm diameter, a 4.0 mm hole size, 207 holes, and a 10 mm thickness at the tip, and the outside diameter of the screw shaft was 86 mm. While the screw shaft rotation speed of the meat chopper was set to 130 rpm, the hydrogel (1) and water vapor were simultaneously supplied to the meat chopper at 4640 [g/min] and 83 [g/min], respectively. Thus, gel-grinding was performed on the hydrogel (1). As a result, a ground gel after gel-grinding, that is, a particulate hydrogel (1) was obtained. The gel-grinding energy (GGE) in the gel-grinding was 29.3 [J/g].

The particulate hydrogel (1) was spread on a 50-mesh wire net and dried with hot air at 180°C for 30 minutes to provide a dried product A(1). The dried product A(1) was ground with a roller mill (WML type roller grinder, available from Inokuchi Giken Kogyosho) to provide a ground product (1). Further, the ground product (1) was sieved with JIS sieves having mesh sizes of 850 $\mu$m, 600 $\mu$m, 500 $\mu$m, 300 $\mu$m, 150 $\mu$m, and 45 $\mu$m.

As a result, water-absorbent resin particles (1) having an irregularly crushed shape were obtained. The physical properties of the water-absorbent resin particles (1) were measured by the method described above. As a result, the water-absorbent resin particles (1) had a mass-average particle size (D50) of 358 $\mu$m, a logarithmic standard deviation ($\sigma\zeta$) of particle size distribution of 0.36, and a centrifuge retention capacity (CRC) of 53.4 (g/g).

Production Example 2

Instead of 0.44 parts by weight of PEGDA-9, 0.36 parts by weight of polyethylene glycol diacrylate having an average n number of 4 was used. In addition, 0.82 parts by weight of a 45 wt.% diethylenetriamine pentaacetic acid aqueous solution was used instead of 1.18 parts by weight of the 31 wt.% ethylenediaminetetramethylenephosphonic acid aqueous solution. Except for these, the same method as in Production Example 1 was performed to provide water-absorbent resin particles having an irregularly crushed shape. The water-absorbent resin particles are referred to as water-absorbent resin particles (2). The gel-grinding energy (GGE) in the gel-grinding of Production Example 2 was 27.9 [J/g]. The physical properties of the water-absorbent resin particles (2) were measured by the method described above. As a result, the water-absorbent resin particles (2) had a mass-average particle size (D50) of 337 $\mu$m, a logarithmic standard deviation ($\sigma\zeta$) of particle size distribution of 0.34, and a centrifuge retention capacity (CRC) of 56.1 (g/g).

Production Example 3

The amount of PEGDA-9 used was changed to 0.52 parts by weight. In addition, 1.83 parts by weight of a 1 wt.% diethylenetriamine pentaacetic acid aqueous solution was used instead of 1.18 parts by weight of the 31 wt.% ethylenediaminetetramethylenephosphonic acid aqueous solution. Further, the amount of deionized water used was changed to 332.9 parts by weight. Except for these, the same method as in Production Example 1 was performed to provide water-absorbent resin particles having an irregularly crushed shape. The water-absorbent resin particles are referred to as water-absorbent resin particles (3). The gel-grinding energy (GGE) in the gel-grinding of Production Example 3 was 31.1 [J/g]. The physical properties of the water-absorbent resin particles (3) were measured by the method described above. As a result, the water-absorbent resin particles (3) had a mass-average particle size (D50) of 346 $\mu$m, a logarithmic standard deviation ($\sigma\zeta$) of particle size distribution of 0.35, and a centrifuge retention capacity (CRC) of 51.1 (g/g).

Production Example 4

The water-absorbent resin particles having an irregularly crushed shape were obtained in the same manner as in Production Example 1 except that the hole diameter of the perforated plate at the tip of the screw extruder was changed to a hole diameter of 9.5 mm and the number of holes was changed to 40. The water-absorbent resin particles are referred to as water-absorbent resin particles (4). The gel-grinding energy (GGE) in the gel-grinding of Production Example 4 was 8.3 [J/g]. The physical properties of the water-absorbent resin particles (4) were measured by the method described above. As a result, the water-absorbent resin particles (4) had a mass-average particle size (D50) of 341 $\mu$m, a logarithmic standard deviation ($\sigma\zeta$) of particle size distribution of 0.34, and a centrifuge retention capacity (CRC) of 53.7 (g/g).

Production Example 5

The water-absorbent resin particles having an irregularly crushed shape were obtained in the same manner as in Production Example 2 except that the hole diameter of the perforated plate at the tip of the screw extruder was changed to a hole diameter of 9.5 mm and the number of holes was changed to 40. The water-absorbent resin particles are referred to as water-absorbent resin particles (5). The gel-grinding energy (GGE) in the gel-grinding of Production Example 5 was 7.2 [J/g]. The physical properties of the water-absorbent resin particles (5) were measured by the method described above. As a result, the water-absorbent resin particles (5) had a mass-average particle size (D50) of 353 $\mu$m, a logarithmic standard deviation ($\sigma\zeta$) of particle size distribution of 0.36, and a centrifuge retention capacity (CRC) of 56.3 (g/g).

Production Example 6

The water-absorbent resin particles having an irregularly crushed shape were obtained in the same manner as in Production Example 3 except that the hole diameter of the perforated plate at the tip of the screw extruder was changed to a hole diameter of 9.5 mm and the number of holes was changed to 40. The water-absorbent resin particles are referred to as water-absorbent resin particles (6). The gel-grinding energy (GGE) in the gel-grinding of Production Example 6 was 9.8 [J/g]. The physical properties of the water-absorbent resin particles (6) were measured by the method described above. As a result, the water-absorbent resin particles (6) had a mass-average particle size (D50) of 338 $\mu$m, a logarithmic standard deviation ($\sigma\zeta$) of particle size distribution of 0.34, and a centrifuge retention capacity (CRC) of 50.9 (g/g).

Production Example 7

Water-absorbent resin particles (7) were produced with reference to Example 12-1 of WO 2017/170605.

Production Example 8

Water-absorbent resin particles (8) were produced with reference to Example 1-13 of WO 2021/201177.

Production Example 9

Water-absorbent resin particles (9) having an irregularly crushed shape were obtained in the same manner as in Production Example 1 except for the following changes.
The amount of PEGDA-9 used was changed to 0.63 parts by weight. In addition, 1.83 parts by weight of a 1 wt.% diethylenetriamine pentaacetic acid aqueous solution was used instead of 1.18 parts by weight of the 31 wt.% ethylenediaminetetramethylenephosphonic acid aqueous solution. Further, 1.83 parts by weight of a 60 wt.% sodium lactate aqueous solution was added as a raw material of the aqueous monomer solution (1), and the amount of deionized water used was changed to 328.3 parts by weight.
The gel-grinding energy (GGE) in the gel-grinding of Production Example 9 was 29.0 [J/g]. The physical properties of the water-absorbent resin particles (9) were measured by the method described above. The water-absorbent resin particles (9) had a mass-average particle size (D50) of 350 $\mu$m, a logarithmic standard deviation ($\sigma\zeta$) of particle size distribution of 0.37, and a centrifuge retention capacity (CRC) of 53.1 (g/g).

Production Example 10

Water-absorbent resin particles (10) having an irregularly crushed shape were obtained in the same manner as in Production Example 1 except for the following changes.
The amount of PEGDA-9 used was changed to 0.50 parts by weight. In addition, 1.83 parts by weight of a 1 wt.% diethylenetriamine pentaacetic acid aqueous solution was used instead of 1.18 parts by weight of the 31 wt.% ethylenediaminetetramethylenephosphonic acid aqueous solution. Further, 2.93 parts by weight of a 50 wt.% malic acid aqueous solution was added as a raw material of the aqueous monomer solution (1), and the amount of deionized water used was changed to 327.1 parts by weight.
The gel-grinding energy (GGE) in the gel-grinding of Production Example 10 was 28.1 [J/g]. The physical properties of the water-absorbent resin particles (10) were measured by the method described above. The water-absorbent resin particles (10) had a mass-average particle size (D50) of 342 $\mu$m, a logarithmic standard deviation ($\sigma\zeta$) of particle size distribution of 0.36, and a centrifuge retention capacity (CRC) of 54.0 (g/g).

Example 1

The surface-crosslinking agent solution was uniformly mixed with the water-absorbent resin particles (1) obtained in Production Example 1, and then the obtained mixture was heat-treated at 190°C for 30 minutes to provide a water-absorbent resin (1). The surface-crosslinking agent solution is a solution including 0.025 parts by weight of ethylene glycol diglycidyl ether, 0.3 parts by weight of ethylene carbonate, 0.5 parts by weight of propylene glycol, and 2.0 parts by weight of deionized water, per 100 parts by weight of the water-absorbent resin particles (1).
Thereafter, 30 g of the cooled water-absorbent resin (1) was placed in a glass container having a 6 cm diameter and a 11 cm height, and the container was placed in a paint shaker (No. 488 test disperser, available from Toyo Seiki Seisaku-sho, Ltd.). Subsequently, the paint shaker was shaken at 800 (cycle/min) for 30 minutes. Thereafter, the EDTMP•5Na aqueous solution was uniformly mixed with the shaken water-absorbent resin (1) per 100 parts by weight of the water-absorbent resin (1). The EDTMP•5Na aqueous solution was an aqueous solution including 1 part by weight of water and 0.01 parts by weight of sodium ethylenediaminetetramethylenephosphonate (EDTMP•5Na), per 100 parts by weight of the water-absorbent resin (1).

Subsequently, an aqueous solution containing, per 100 parts by weight of the water-absorbent resin (1), 1.0 part by weight of water, 1.0 part by weight of propylene glycol, and 1.5 parts by weight of an aqueous cationic colloidal silica solution was uniformly mixed with the water-absorbent resin (1) in which the EDTMP•5Na aqueous solution was mixed. The aqueous cationic colloidal silica solution is a 30% aqueous solution of Klebosol 30CAL25 (trade name), available from AZ Electronic Materials S.A., which is silicon dioxide fine particles containing aluminum cations.

The resulting mixture was dried at 60°C for 1 hour, and then passed through a JIS standard sieve with an opening of 850 μm to provide a particulate water-absorbing agent (1). The physical properties of the particulate water-absorbing agent (1) were measured by the above-described method. The results are shown in Table 2 below.

Example 2

A particulate water-absorbing agent (2) was obtained in the same operation as in Example 1 except for the following points.

• As the surface-crosslinking agent solution, a solution including 0.025 parts by weight of ethylene glycol diglycidyl ether, 0.31 parts by weight of 1,4-butanediol, 0.5 parts by weight of propylene glycol, and 2 parts by weight of deionized water, per 100 parts by weight of the water-absorbent resin particles (2), was used.
• An aqueous solution including 1 part by weight of water, 0.03 parts by weight of EDTMP•5Na and 0.05 parts by weight of sodium hydrogen sulfite was used instead of the aqueous solution of EDTMP•5Na.
• A solution including 1.0 part by weight of water and 1.5 parts by weight of an aqueous alumina sol solution was used instead of an aqueous solution including 1.0 part by weight of water, 1.0 part by weight of propylene glycol, and 1.5 parts by weight of an aqueous cationic colloidal silica solution.

The physical properties of the particulate water-absorbing agent (2) were measured by the above-described method. The results are shown in Table 2 below. As the aqueous alumina sol solution, trade name: Alumina sol 520-A (20.5% aqueous solution of aluminum oxide, available from Nissan Chemical Corporation) was used.

Example 3

A particulate water-absorbing agent (3) was obtained in the same operation as in Example 1 except for the following points.

• As the surface-crosslinking agent solution, a solution including 0.02 parts by weight of ethylene glycol diglycidyl ether, 1.3 parts by weight of propylene glycol, and 3.2 parts by weight of deionized water per 100 parts by weight of the water-absorbent resin particles (1) was used.
• A mixture obtained by uniformly mixing the water-absorbent resin particles (1) and the surface-crosslinking agent was subjected to a heat treatment at 100°C for 40 minutes instead of a heat treatment at 190°C for 30 minutes.
• The shaking time of the paint shaker was changed from 30 minutes to 10 minutes.
• A polyethylene glycol aqueous solution including 1 part by weight of water and 0.03 parts by weight of polyethylene glycol (average molecular weight: 400) was used instead of the EDTMP•5Na aqueous solution.
• Without using an aqueous solution including 1.0 part by weight of water, 1.0 part by weight of propylene glycol, and 1.5 parts by weight of an aqueous cationic colloidal silica solution, the mixture of the polyethylene glycol aqueous solution and the water-absorbent resin was dried at 60°C for 1 hour and then passed through a JIS standard sieve having a mesh size of 850 μm.
• After passing the mixture through a JIS standard sieve, 0.3 parts by weight of silicon dioxide (trade name: Aerosil 200, available from NIPPON AEROSIL CO., LTD.) was further uniformly mixed.

The physical properties of the particulate water-absorbing agent (3) were measured by the above-described method. The results are shown in Table 2 below.

Example 4

A particulate water-absorbing agent (4) was obtained in the same operation as in Example 1 except for the following points.

• As a surface-crosslinking agent solution, a solution including 0.02 parts by weight of ethylene glycol diglycidyl ether, 1.8 parts by weight of propylene glycol, 2.7 parts by weight of deionized water, and 0.5 parts by weight of aluminum sulfate tetradeca- to octadecahydrate per 100 parts by weight of the water-absorbent resin particles (1) was used.
• A mixture obtained by uniformly mixing the water-absorbent resin particles (1) and the surface-crosslinking agent was subjected to a heat treatment at 100°C for 40 minutes instead of a heat treatment at 190°C for 30 minutes.
• The shaking time of the paint shaker was changed from 30 minutes to 10 minutes.
• Instead of the EDTMP•5Na aqueous solution, a polyethylene glycol aqueous solution including 1 part by weight of water, 0.12 parts by weight of polyethylene glycol (average molecular weight 600), and 0.03 parts by weight of sodium sulfite was used.
• Without using an aqueous solution including 1.0 part by weight of water, 1.0 part by weight of propylene glycol, and 1.5 parts by weight of an aqueous cationic colloidal silica solution, the mixture of the polyethylene glycol aqueous solution and the water-absorbent resin was dried at 60°C for 1 hour and then passed through a JIS standard sieve having a mesh size of 850 μm.

The physical properties of the particulate water-absorbing agent (4) were measured by the above-described method. The results are shown in Table 2 below.

Example 5

A particulate water-absorbing agent (5) was obtained in the same operation as in Example 1 except for the following points.

• As a surface-crosslinking agent solution, a solution including 0.025 parts by weight of ethylene glycol diglycidyl ether, 0.26 parts by weight of 1,3-propanediol, 0.5 parts by weight of propylene glycol, and 2 parts by weight of deionized water per 100 parts by weight of the water-absorbent resin particles (1), was used.
• A polyethylene glycol aqueous solution including 1 part by weight of water and 0.1 parts by weight of polyethylene glycol (average molecular weight: 400) was used instead of the EDTMP•5Na aqueous solution.
• Instead of the aqueous solution including 1.0 part by weight of water, 1.0 part by weight of propylene glycol, and 1.5 parts by weight of an aqueous cationic colloidal silica solution, an aqueous aluminum sulfate solution including 1.5 parts by weight of water and 0.5 parts by weight of aluminum sulfate 14-18 hydrate was used.

The physical properties of the particulate water-absorbing agent (5) were measured by the above-described method. The results are shown in Table 2 below.

Example 6

A particulate water-absorbing agent (6) was obtained in the same operation as in Example 1 except for the following points.
The water-absorbent resin particles (2) prepared in Production Example 2 were used instead of the water-absorbent resin particles (1).

• Instead of the EDTMP•5Na aqueous solution, a DTPA•3Na aqueous solution including 2.0 parts by weight of water, 0.02 parts by weight of diethylenetriaminepentaacetic acid•trisodium (DTPA•3Na), and 0.1 parts by weight of sodium sulfite was used.
• Without using an aqueous solution including 1.0 part by weight of water, 1.0 part by weight of propylene glycol, and 1.5 parts by weight of an aqueous cationic colloidal silica solution, the mixture of the DTPA•3Na aqueous solution and the water-absorbent resin was dried at 60°C for 1 hour and then passed through a JIS standard sieve having a mesh size of 850 μm.
• After passing the mixture through a JIS standard sieve, 0.3 parts by weight of hydrotalcite (trade name: DHT-6, available from Kyowa Chemical Industry Co., Ltd.) was further mixed uniformly.

The physical properties of the particulate water-absorbing agent (6) were measured by the above-described method. The results are shown in Table 2 below.

Example 7

A particulate water-absorbing agent (7) was obtained in the same operation as in Example 1 except for the following points.

• The water-absorbent resin particles (2) prepared in Production Example 2 were used instead of the water-absorbent resin particles (1).

• As the surface-crosslinking agent solution, a solution including 0.02 parts by weight of ethylene glycol diglycidyl ether, 1.8 parts by weight of propylene glycol, and 2.7 parts by weight of deionized water per 100 parts by weight of the water-absorbent resin particles (2) was used.

• A mixture obtained by uniformly mixing the water-absorbent resin particles (2) and the surface-crosslinking agent was subjected to a heat treatment at 100°C for 40 minutes instead of a heat treatment at 190°C for 30 minutes.

• The shaking time of the paint shaker was changed from 30 minutes to 10 minutes.

• Instead of the EDTMP•5Na aqueous solution, an aqueous sodium hydrogen sulfite solution including 0.5 parts by weight of water and 0.03 parts by weight of sodium hydrogen sulfite was used.

• Instead of an aqueous solution including 1.0 part by weight of water, 1.0 part by weight of propylene glycol, and 1.5 parts by weight of an aqueous cationic colloidal silica solution, a solution including 1.0 part by weight of water, 1.0 part by weight of propylene glycol, and 1.5 parts by weight of an aqueous alumina sol solution was used.

The physical properties of the particulate water-absorbing agent (7) were measured by the above-described method. The results are shown in Table 2 below. As the aqueous alumina sol solution, trade name: Alumina sol 520-A (20.5% aqueous solution of aluminum oxide, available from Nissan Chemical Corporation) was used.

Example 8

A particulate water-absorbing agent (8) was obtained in the same operation as in Example 1 except for the following points.

• The water-absorbent resin particles (2) prepared in Production Example 2 were used instead of the water-absorbent resin particles (1).

• As the surface-crosslinking agent solution, a solution including 0.025 parts by weight of ethylene glycol diglycidyl ether, 0.31 parts by weight of 1,4-butanediol, 0.5 parts by weight of propylene glycol, and 2 parts by weight of deionized water, per 100 parts by weight of the water-absorbent resin particles (2), was used.

• The shaking time of the paint shaker was changed from 30 minutes to 10 minutes.

• Without using an EDTMP•5Na aqueous solution, a mixture of an aqueous solution including 1.0 part by weight of water, 1.0 part by weight of propylene glycol, and 1.5 parts by weight of an aqueous cationic colloidal silica solution and a water-absorbent resin was dried at 60°C for 1 hour, and then passed through a JIS standard sieve having an opening of 850 μm.

The physical properties of the particulate water-absorbing agent (8) were measured by the above-described method. The results are shown in Table 2 below.

Example 9

A particulate water-absorbing agent (9) was obtained in the same operation as in Example 1 except for the following points.

• The water-absorbent resin particles (2) prepared in Production Example 2 were used instead of the water-absorbent resin particles (1).

• Instead of the EDTMP•5Na aqueous solution, a DTPA•3Na aqueous solution including 1.5 parts by weight of water and 0.01 parts by weight of diethylenetriaminepentaacetic acid•trisodium (DTPA•3Na) was used.

• Instead of an aqueous solution including 1.0 part by weight of water, 1.0 part by weight of propylene glycol, and 1.5 parts by weight of an aqueous cationic colloidal silica solution, an aqueous aluminum sulfate solution including 1.5 parts by weight of water, 1.0 part by weight of propylene glycol, and 0.5 parts by weight of aluminum sulfate 14-18 hydrate was used.

The physical properties of the particulate water-absorbing agent (9) were measured by the above-described method. The results are shown in Table 2 below.

Example 10

A particulate water-absorbing agent (10) was obtained in the same operation as in Example 1 except for the following points.

• The water-absorbent resin particles (2) prepared in Production Example 2 were used instead of the water-absorbent resin particles (1).

• Instead of the EDTMP•5Na aqueous solution, a mixed solution including 2.0 parts by weight of water, 0.01 parts by weight of trisodium diethylenetriaminepentaacetate (DTPA•3Na), 0.05 parts by weight of sodium sulfite, and 0.03 parts by weight of polyethylene glycol (average molecular weight 600) was used.

• Without using an aqueous solution including 1.0 part by weight of water, 1.0 part by weight of propylene glycol, and 1.5 parts by weight of an aqueous cationic colloidal silica solution, a mixture of the mixed solution and a water-absorbent resin was dried at 60°C for 1 hour, and then passed through a JIS standard sieve having an opening of 850 μm.

• After passing the mixture through a JIS standard sieve, 0.3 parts by weight of silicon dioxide (trade name: REOLOSIL QS-20, available from Tokuyama Corporation) was further mixed uniformly.

The physical properties of the particulate water-absorbing agent (10) were measured by the above-described method. The results are shown in Table 2 below.

Example 11

A particulate water-absorbing agent (11) was obtained in the same operation as in Example 1 except for the following points.

• The water-absorbent resin particles (3) prepared in Production Example 3 were used instead of the water-absorbent resin particles (1).

• As a surface-crosslinking agent solution, a solution including 0.02 parts by weight of ethylene glycol diglycidyl ether, 2.45 parts by weight of propylene glycol, 3.55 parts by weight of deionized water, and 0.5 parts by weight of aluminum sulfate 14-18 hydrate per 100 parts by weight of the water-absorbent resin particles (3) was used.

• A mixture obtained by uniformly mixing the water-absorbent resin particles (3) and the surface-crosslinking agent was subjected to a heat treatment at 100°C for 40 minutes instead of a heat treatment at 190°C for 30 minutes.

• The shaking time of the paint shaker was changed from 30 minutes to 10 minutes.

• Instead of the EDTMP•5Na aqueous solution, an aqueous solution of DTPA•3Na including 1.5 parts by weight of water, 0.15 parts by weight of polyethylene glycol (average molecular weight 400), and 0.03 parts by weight of diethylenetriaminepentaacetic acid•trisodium (DTPA•3Na) was used.

• Without using an aqueous solution including 1.0 part by weight of water, 1.0 part by weight of propylene glycol, and 1.5 parts by weight of an aqueous cationic colloidal silica solution, the mixture of the DTPA•3Na aqueous solution and the water-absorbent resin was dried at 60°C for 1 hour and then passed through a JIS standard sieve having a mesh size of 850 μm.

The physical properties of the particulate water-absorbing agent (11) were measured by the above-described method. The results are shown in Table 2 below.

Example 12

A particulate water-absorbing agent (12) was obtained in the same operation as in Example 1 except for the following points.

• The water-absorbent resin particles (3) prepared in Production Example 3 were used instead of the water-absorbent resin particles (1).

• As a surface-crosslinking agent solution, a solution including 0.025 parts by weight of ethylene glycol diglycidyl ether, 0.26 parts by weight of 1,3-propanediol, 0.5 parts by weight of propylene glycol, and 2 parts by weight of deionized water per 100 parts by weight of the water-absorbent resin particles (3), was used.

• Instead of the EDTMP•5Na aqueous solution, a mixed solution including 1.5 parts by weight of water, 0.05 parts by weight of polyethylene glycol (average molecular weight 600), 0.05 parts by weight of the sodium salt of ethylenediaminetetramethylenephosphonic acid (EDTMP•5Na), and 0.1 parts by weight of sodium sulfite was used.

• Instead of an aqueous solution including 1.0 part by weight of water, 1.0 part by weight of propylene glycol, and 1.5 parts by weight of an aqueous cationic colloidal silica solution, a solution including 1.0 part by weight of water, 1.0 part by weight of propylene glycol, and 1.5 parts by weight of an aqueous alumina sol solution was used.

The physical properties of the particulate water-absorbing agent (12) were measured by the above-described method. The results are shown in Table 2 below.

Example 13

A particulate water-absorbing agent (13) was obtained in the same operation as in Example 1 except for the following points.

• The water-absorbent resin particles (3) prepared in Production Example 3 were used instead of the water-absorbent resin particles (1).
• As the surface-crosslinking agent solution, a solution including 0.02 parts by weight of ethylene glycol diglycidyl ether, 1.3 parts by weight of propylene glycol, and 3.2 parts by weight of deionized water per 100 parts by weight of the water-absorbent resin particles (3) was used.
• A mixture obtained by uniformly mixing the water-absorbent resin particles (3) and the surface-crosslinking agent was subjected to a heat treatment at 100°C for 40 minutes instead of a heat treatment at 190°C for 30 minutes.
• Instead of the EDTMP•5Na aqueous solution, a DTPA•3Na aqueous solution including 1 part by weight of water and 0.02 parts by weight of diethylenetriaminepentaacetic acid•trisodium (DTPA•3Na) was used.
• Instead of an aqueous solution including 1.0 part by weight of water, 1.0 part by weight of propylene glycol, and 1.5 parts by weight of an aqueous cationic colloidal silica solution, an aqueous solution including 1.0 part by weight of water and 1.5 parts by weight of an aqueous cationic colloidal silica solution was used.

The physical properties of the particulate water-absorbing agent (13) were measured by the above-described method. The results are shown in Table 2 below.

Comparative Example 1

A comparative particulate water-absorbing agent (1) was produced in the same manner as in Example 1 except that the water-absorbent resin particles (4) prepared in Production Example 4 were used instead of the water-absorbent resin particles (1). The physical properties of the comparative particulate water-absorbing agent (1) were measured by the above-described method. The results are shown in Table 2 below.

Comparative Example 2

A comparative particulate water-absorbing agent (2) was produced in the same manner as in Example 3 except that the water-absorbent resin particles (4) prepared in Production Example 4 were used instead of the water-absorbent resin particles (1). The physical properties of the comparative particulate water-absorbing agent (2) were measured by the above-described method. The results are shown in Table 2 below.

Comparative Example 3

A comparative particulate water-absorbing agent (3) was produced in the same manner as in Example 5 except that the water-absorbent resin particles (4) prepared in Production Example 4 were used instead of the water-absorbent resin particles (1). The physical properties of the comparative particulate water-absorbing agent (3) were measured by the above-described method. The results are shown in Table 2 below.

Comparative Example 4

A comparative particulate water-absorbing agent (4) was produced in the same manner as in Example 6 except that the water-absorbent resin particles (5) prepared in Production Example 5 were used instead of the water-absorbent resin particles (2). The physical properties of the comparative particulate water-absorbing agent (4) were measured by the above-described method. The results are shown in Table 2 below.

Comparative Example 5

A comparative particulate water-absorbing agent (5) was produced in the same manner as in Example 9 except that the water-absorbent resin particles (5) prepared in Production Example 5 were used instead of the water-absorbent resin particles (2). The physical properties of the comparative particulate water-absorbing agent (5) were measured by the above-described method. The results are shown in Table 2 below.

Comparative Example 6

A comparative particulate water-absorbing agent (6) was produced in the same manner as in Example 12 except that the water-absorbent resin particles (6) prepared in Production Example 6 were used instead of the water-absorbent resin particles (3). The physical properties of the comparative particulate water-absorbing agent (6) were measured by the above-described method. The results are shown in Table 2 below.

Comparative Example 7

A comparative particulate water-absorbing agent (7) was produced in the same manner as in Example 7 of WO 2017/170605 A. The physical properties of the comparative particulate water-absorbing agent (7) were measured by the above-described method. The results are shown in Table 2 below.

Comparative Example 8

A comparative particulate water-absorbing agent (8) was produced in the same manner as in Example 12-1 of WO 2017/170605 A. The physical properties of the comparative particulate water-absorbing agent (8) were measured by the above-described method. The results are shown in Table 2 below.

Comparative Example 9

A comparative particulate water-absorbing agent (9) was produced in the same manner as in Example 1-13 of WO 2021/201177 A. The physical properties of the comparative particulate water-absorbing agent (9) were measured by the above-described method. The results are shown in Table 2 below.

Example 14

A particulate water-absorbing agent (14) was obtained in the same manner as in Example 1 except for the following points.

• The water-absorbent resin particles (9) prepared in Production Example 9 were used instead of the water-absorbent resin particles (1).
• As the surface-crosslinking agent solution, a solution including 0.025 parts by weight of ethylene glycol diglycidyl ether, 1.8 parts by weight of propylene glycol, and 2.7 parts by weight of deionized water per 100 parts by weight of the water-absorbent resin particles (9) was used.
• A mixture obtained by uniformly mixing the water-absorbent resin particles (9) and the surface-crosslinking agent was subjected to a heat treatment at 100°C for 40 minutes instead of a heat treatment at 190°C for 30 minutes.
• Instead of the EDTMP•5Na aqueous solution, a DTPA•3Na aqueous solution including 1.5 parts by weight of water and 0.02 parts by weight of diethylenetriaminepentaacetic acid•trisodium (DTPA•3Na) was used.
• Instead of an aqueous solution including 1.0 part by weight of water, 1.0 part by weight of propylene glycol, and 1.5 parts by weight of an aqueous cationic colloidal silica solution, an aqueous solution including 1.0 part by weight of water, 1.0 part by weight of propylene glycol, and 1.5 parts by weight of an aqueous alumina sol solution was used.

The physical properties of the particulate water-absorbing agent (14) were measured by the above-described method. The results are shown in Table 2 below. As the aqueous alumina sol solution, trade name: Alumina sol 520-A (20.5% aqueous solution of aluminum oxide, available from Nissan Chemical Corporation) was used.

Example 15

A particulate water-absorbing agent (15) was obtained in the same operation as in Example 1 except for the following points.

• The water-absorbent resin particles (9) prepared in Production Example 9 were used instead of the water-absorbent resin particles (1).
• As a surface-crosslinking agent solution, a solution including 0.025 parts by weight of ethylene glycol diglycidyl ether, 0.26 parts by weight of 1,3-propanediol, 0.5 parts by weight of propylene glycol, and 2 parts by weight of deionized water per 100 parts by weight of the water-absorbent resin particles (9), was used.
• Instead of the EDTMP•5Na aqueous solution, a mixed solution including 0.5 parts by weight of water, 0.05 parts by weight of polyethylene glycol (average molecular weight 400), 0.03 parts by weight of the sodium salt of ethylenediaminetetramethylenephosphonic acid (EDTMP•5Na), and 0.05 parts by weight of sodium sulfite was used.

• Instead of an aqueous solution including 1.0 part by weight of water, 1.0 part by weight of propylene glycol, and 1.5 parts by weight of an aqueous cationic colloidal silica solution, an aqueous solution including 1.5 parts by weight of water, 1.0 part by weight of propylene glycol, and 0.5 parts by weight of aluminum sulfate 14-18 hydrate was used.

The physical properties of the particulate water-absorbing agent (15) were measured by the above-described method. The results are shown in Table 2 below.

Example 16

A particulate water-absorbing agent (16) was obtained in the same operation as in Example 1 except for the following points.

• The water-absorbent resin particles (9) prepared in Production Example 9 were used instead of the water-absorbent resin particles (1).

• As the surface-crosslinking agent solution, a solution including 0.02 parts by weight of ethylene glycol diglycidyl ether, 1.3 parts by weight of propylene glycol, and 3.2 parts by weight of deionized water per 100 parts by weight of the water-absorbent resin particles (9) was used.

• A mixture obtained by uniformly mixing the water-absorbent resin particles (9) and the surface-crosslinking agent was subjected to a heat treatment at 100°C for 40 minutes instead of a heat treatment at 190°C for 30 minutes.

• The shaking time of the paint shaker was changed from 30 minutes to 10 minutes.

• Instead of the EDTMP•5Na aqueous solution, an aqueous solution including 1.5 parts by weight of water, 0.07 parts by weight of the sodium salt of ethylenediaminetetramethylenephosphonic acid (EDTMP•5Na), and 0.1 parts by weight of sodium sulfite was used.

• Without using an aqueous solution including 1.0 part by weight of water, 1.0 part by weight of propylene glycol, and 1.5 parts by weight of an aqueous cationic colloidal silica solution, a mixture of the aqueous solution and a water-absorbent resin was dried at 60°C for 1 hour, and then passed through a JIS standard sieve having an opening of 850 μm.

• After passing the mixture through a JIS standard sieve, 0.3 parts by weight of hydrotalcite (trade name: DHT-6, available from Kyowa Chemical Industry Co., Ltd.) was further mixed uniformly.

The physical properties of the particulate water-absorbing agent (16) were measured by the above-described method. The results are shown in Table 2 below.

Example 17

A particulate water-absorbing agent (17) was obtained in the same operation as in Example 1 except for the following points.

• The water-absorbent resin particles (10) prepared in Production Example 10 were used instead of the water-absorbent resin particles (1).

• As the surface-crosslinking agent solution, a solution including 0.025 parts by weight of ethylene glycol diglycidyl ether, 1.3 parts by weight of propylene glycol, and 3.2 parts by weight of deionized water per 100 parts by weight of the water-absorbent resin particles (10) was used.

• A mixture obtained by uniformly mixing the water-absorbent resin particles (10) and the surface-crosslinking agent was subjected to a heat treatment at 100°C for 40 minutes instead of a heat treatment at 190°C for 30 minutes.

• Instead of the EDTMP•5Na aqueous solution, a mixed solution including 1.5 parts by weight of water, 0.03 parts by weight of polyethylene glycol (average molecular weight 600), and 0.05 parts by weight of diethylenetriaminepentaacetic acid•trisodium salt (DTPA•3Na) was used.

• Instead of an aqueous solution including 1.0 part by weight of water, 1.0 part by weight of propylene glycol, and 1.5 parts by weight of an aqueous cationic colloidal silica solution, an aqueous solution including 1.0 part by weight of water and 1.5 parts by weight of an aqueous cationic colloidal silica solution was used.

The physical properties of the particulate water-absorbing agent (17) were measured by the above-described method. The results are shown in Table 2 below.

Example 18

A particulate water-absorbing agent (18) was obtained in the same operation as in Example 1 except for the following points.

• The water-absorbent resin particles (10) prepared in Production Example 10 were used instead of the water-absorbent resin particles (1).
• As the surface-crosslinking agent solution, a solution including 0.02 parts by weight of ethylene glycol diglycidyl ether, 1.8 parts by weight of propylene glycol, and 2.7 parts by weight of deionized water per 100 parts by weight of the water-absorbent resin particles (10) was used.
• A mixture obtained by uniformly mixing the water-absorbent resin particles (10) and the surface-crosslinking agent was subjected to a heat treatment at 100°C for 40 minutes instead of a heat treatment at 190°C for 30 minutes.
• The shaking time of the paint shaker was changed from 30 minutes to 10 minutes.
• Instead of the EDTMP•5Na aqueous solution, a mixed solution including 2 parts by weight of water, 0.25 parts by weight of polyethylene glycol (average molecular weight 400), and 0.1 parts by weight of diethylenetriaminepentaacetic acid•trisodium salt (DTPA•3Na) was used.
• Instead of the aqueous solution including 1.0 part by weight of water, 1.0 part by weight of propylene glycol, and 1.5 parts by weight of an aqueous cationic colloidal silica solution, an aqueous aluminum sulfate solution including 1.5 parts by weight of water and 0.5 parts by weight of aluminum sulfate 14-18 hydrate was used.

The physical properties of the particulate water-absorbing agent (18) were measured by the above-described method. The results are shown in Table 2 below.

Example 19

A particulate water-absorbing agent (19) was obtained in the same manner as in Example 1 except for the following points.
The water-absorbent resin particles (10) prepared in Production Example 10 were used instead of the water-absorbent resin particles (1).

• As a surface-crosslinking agent solution, a solution including 0.02 parts by weight of ethylene glycol diglycidyl ether, 1.8 parts by weight of propylene glycol, 2.7 parts by weight of deionized water, and 0.5 parts by weight of aluminum sulfate 14-18 hydrate per 100 parts by weight of the water-absorbent resin particles (10) was used.
• A mixture obtained by uniformly mixing the water-absorbent resin particles (10) and the surface-crosslinking agent was subjected to a heat treatment at 100°C for 40 minutes instead of a heat treatment at 190°C for 30 minutes.
• Instead of the EDTMP•5Na aqueous solution, a mixed solution including 2 parts by weight of water, 0.2 parts by weight of polyethylene glycol (average molecular weight 600), 0.07 parts by weight of trisodium diethylenetriaminepentaacetate (DTPA•3Na), and 0.1 parts by weight of sodium sulfite was used.
• Without using an aqueous solution including 1.0 part by weight of water, 1.0 part by weight of propylene glycol, and 1.5 parts by weight of an aqueous cationic colloidal silica solution, a mixture of the mixed solution and a water-absorbent resin was dried at 60°C for 1 hour, and then passed through a JIS standard sieve having an opening of 850 μm.

The physical properties of the particulate water-absorbing agent (19) were measured by the above-described method. The results are shown in Table 2 below.

Example 20

A particulate water-absorbing agent (20) was obtained in the same operation as in Example 1 except for the following points.

• The water-absorbent resin particles (10) prepared in Production Example 10 were used instead of the water-absorbent resin particles (1).
• Instead of the EDTMP•5Na aqueous solution, an aqueous solution including 1 part by weight of water, 0.07 parts by weight of the sodium salt of ethylenediaminetetramethylenephosphonic acid (EDTMP•5Na), and 0.03 parts by weight of sodium hydrogen sulfite was used.
• Without using an aqueous solution including 1.0 part by weight of water, 1.0 part by weight of propylene glycol, and 1.5 parts by weight of an aqueous cationic colloidal silica solution, a mixture of the aqueous solution and a water-absorbent resin was dried at 60°C for 1 hour, and then passed through a JIS standard sieve having an opening of 850 μm.
• After passing the mixture through a JIS standard sieve, 0.3 parts by weight of silicon dioxide (trade name: Aerosil 200, available from NIPPON AEROSIL CO., LTD.) was further uniformly mixed.

The physical properties of the particulate water-absorbing agent (20) were measured by the above-described method. The results are shown in Table 2 below.

Comparative Example 10

The surface-crosslinking agent solution was uniformly mixed with the water-absorbent resin particles (6) obtained in Production Example 6, and then the obtained mixture was heat-treated at 190°C for 30 minutes to provide a water-absorbent resin (1). The surface-crosslinking agent solution was a solution including 0.025 parts by weight of ethylene glycol diglycidyl ether, 0.3 parts by weight of ethylene carbonate, 0.5 parts by weight of propylene glycol, and 2.0 parts by weight of deionized water, per 100 parts by weight of the water-absorbent resin particles (1).
Thereafter, 30 g of the cooled water-absorbent resin (1) was placed in a glass container having a 6 cm diameter and an 11 cm height, and the container was placed in a paint shaker (No. 488 test disperser, available from Toyo Seiki Seisaku-sho, Ltd.). Subsequently, the paint shaker was shaken at 800 (cycle/min) for 30 minutes.
The resulting mixture was dried at 60°C for 1 hour, and then passed through a JIS standard sieve with an opening of 850 μm to provide a comparative particulate water-absorbing agent (10). The physical properties of the comparative particulate water-absorbing agent (10) were measured by the above-described method. The results are shown in Table 2 below.

Comparative Example 11

The aqueous aluminum sulfate solution was uniformly mixed with the comparative particulate water-absorbing agent (10) obtained in Comparative Example 10. The aqueous aluminum sulfate solution was an aqueous solution including 1.5 parts by weight of water and 0.5 parts by weight of aluminum sulfate 14-18 hydrate per 100 parts by weight of the comparative particulate water-absorbing agent (10).
The resulting mixture was dried at 60°C for 1 hour, and then passed through a JIS standard sieve with an opening of 850 μm to provide a comparative particulate water-absorbing agent (11). The physical properties of the comparative particulate water-absorbing agent (11) were measured by the above-described method. The results are shown in Table 2 below.

Comparative Example 12

An aqueous solution including 1 part by weight of water, 0.01 parts by weight of diethylenetriaminepentaacetic acid•trisodium (DTPA•3Na), and 0.004 parts by weight of polyoxyethylene sorbitan monostearate (trade name: Rheodol Tw S120V, available from Kao Corporation) was uniformly mixed with the comparative particulate water-absorbing agent (10) obtained in Comparative Example 10.
Subsequently, the aqueous aluminum sulfate solution was uniformly mixed. The aqueous aluminum sulfate solution was an aqueous solution including 1.5 parts by weight of water and 0.5 parts by weight of aluminum sulfate 14-18 hydrate per 100 parts by weight of the comparative particulate water-absorbing agent (10) .
The resulting mixture was dried at 60°C for 1 hour, and then passed through a JIS standard sieve with an opening of 850 μm to provide a comparative particulate water-absorbing agent (12). The physical properties of the comparative particulate water-absorbing agent (12) were measured by the above-described method. The results are shown in Table 2 below.

Result

Gel-grinding energy (GGE) as production conditions in Production Examples 1 to 10 and physical properties of water-absorbent resin particles (1) to (10) prepared in Production Examples 1 to 10 are shown in Table 1 below.

[Table 1]

Table 1

| | Gel-grinding energy (GGE) [J/g] | CRC [g/g] | Weight-average particle size (D50) [μm] | Logarithmic standard deviation (δζ) of particle size distribution |
|---|---|---|---|---|
| Production Example 1 | 29.3 | 53.4 | 358 | 0.36 |
| Production Example 2 | 27.9 | 56.1 | 337 | 0.34 |
| Production Example 3 | 31.1 | 51.1 | 346 | 0.35 |
| Production Example 4 | 8.3 | 53.7 | 341 | 0.34 |
| Production Example 5 | 7.2 | 56.3 | 353 | 0.36 |
| Production Example 6 | 9.8 | 50.9 | 338 | 0.34 |
| Production Example 7 | 19.6 | 49.4 | 366 | 0.32 |
| Production Example 8 | 10.2 | 50.1 | 298 | 0.35 |
| Production Example 9 | 29.0 | 53.1 | 350 | 0.37 |
| Production Example 10 | 28.1 | 54.0 | 342 | 0.36 |

The physical properties of the particulate water-absorbing agents (1) to (20) produced in Examples 1 to 20 and the comparative particulate water-absorbing agents (1) to (12) in Comparative Examples 1 to 12 are shown below.

[Table 2]

Table 2

| | SRC (NP) [g/g] | SRC (4.83 kPa) [g/g] | CRC [g/g] | Vortex [sec] | STR [mN/m] | Absorption amount of absorbent body [g] | Amount of re-wetting from absorbent body [g] |
|---|---|---|---|---|---|---|---|
| Example 1 | 60.3 | 44.1 | 41.5 | 36 | 71.6 | 227 | 3.1 |
| Example 2 | 61.9 | 43.2 | 41.7 | 36 | 71.7 | 226 | 3.0 |
| Example 3 | 60.9 | 43.5 | 41.5 | 34 | 71.3 | 228 | 3.0 |
| Example 4 | 61.1 | 43.9 | 41.1 | 34 | 70.9 | 227 | 3.2 |
| Example 5 | 57.3 | 43.0 | 41.3 | 39 | 71.2 | 218 | 2.9 |
| Example 6 | 56.4 | 44.0 | 43.2 | 40 | 71.6 | 216 | 3.2 |
| Example 7 | 62.5 | 43.4 | 43.6 | 29 | 71.5 | 230 | 3.4 |
| Example 8 | 60.5 | 44.4 | 44.1 | 34 | 71.6 | 230 | 3.4 |
| Example 9 | 58.2 | 43.2 | 43.5 | 36 | 71.6 | 220 | 3.2 |
| Example 10 | 62.6 | 43.8 | 43.9 | 35 | 71.2 | 231 | 3.0 |
| Example 11 | 59.6 | 42.3 | 40.0 | 32 | 71.3 | 224 | 3.1 |
| Example 12 | 59.9 | 41.9 | 40.4 | 34 | 71.4 | 222 | 3.2 |
| Example 13 | 58.3 | 42.7 | 39.8 | 32 | 71.5 | 219 | 3.2 |
| Example 14 | 59.4 | 41.8 | 40.1 | 32 | 71.9 | 222 | 3.5 |
| Example 15 | 57.3 | 43.1 | 42.0 | 35 | 71.6 | 221 | 3.3 |
| Example 16 | 55.1 | 42.9 | 41.8 | 35 | 71.7 | 216 | 3.2 |
| Example 17 | 59.2 | 43.6 | 41.1 | 34 | 71.6 | 225 | 3.0 |
| Example 18 | 56.9 | 43.2 | 41.9 | 34 | 71.4 | 218 | 3.2 |

(continued)

| | SRC (NP) [g/g] | SRC (4.83 kPa) [g/g] | CRC [g/g] | Vortex [sec] | STR [mN/m] | Absorption amount of absorbent body [g] | Amount of re-wetting from absorbent body [g] |
|---|---|---|---|---|---|---|---|
| Example 19 | 59.0 | 43.0 | 42.1 | 32 | 71.4 | 223 | 3.2 |
| Example 20 | 62.0 | 44.2 | 42.5 | 35 | 71.8 | 231 | 2.9 |
| Comparative Example 1 | 54.3 | 41.1 | 41.1 | 43 | 71.5 | 208 | 5.7 |
| Comparative Example 2 | 53.9 | 39.9 | 40.7 | 42 | 71.4 | 206 | 5.7 |
| Comparative Example 3 | 54.1 | 40.6 | 41.0 | 45 | 71.0 | 206 | 5.5 |
| Comparative Example 4 | 49.8 | 41.5 | 43.9 | 53 | 71.6 | 203 | 5.6 |
| Comparative Example 5 | 52.2 | 41.1 | 44.1 | 49 | 71.7 | 204 | 5.7 |
| Comparative Example 6 | 53.8 | 39.0 | 39.9 | 43 | 71.3 | 205 | 5.4 |
| Comparative Example 7 | 58.4 | 41.3 | 39.5 | 42 | 72.0 | 212 | 4.9 |
| Comparative Example 8 | 54.0 | 41.1 | 39.5 | 42 | 72.0 | 209 | 4.8 |
| Comparative Example 9 | 53.5 | 35.7 | 36.5 | 38 | 71.2 | 200 | 7.8 |
| Comparative Example 10 | 47.1 | 38.2 | 40.3 | 52 | 71.9 | 193 | 5.5 |
| Comparative Example 11 | 51.3 | 39.3 | 40.1 | 47 | 71.8 | 205 | 5.4 |
| Comparative Example 12 | 50.9 | 39.2 | 39.4 | 47 | 58.0 | 203 | 6.1 |

As shown in Table 2, the particulate water-absorbing agents (1) to (20) produced in Examples 1 to 20 have SRC (NP) exceeding 50.0 g/g and SRC (4.83 kPa) exceeding 41.5 g/g. Therefore, the particulate water-absorbing agents (1) to (20) correspond to the particulate water-absorbing agents of the present invention. In addition, the particulate water-absorbing agents (1) to (20) are excellent in water absorption speed (Vortex). In contrast, the comparative particulate water-absorbing agents (1) to (12) produced in Comparative Examples 1 to 12 have an SRC (NP) of 50.0 g/g or less and/or an SRC (4.83 kPa) of 41.5 or less, and do not correspond to the particulate water-absorbing agent of the present invention.

In addition, the absorbent body with the particulate water-absorbing agents (1) to (20) used has a larger absorption amount in the absorbent body and a smaller amount of re-wetting in the absorbent body than the comparative particulate water-absorbing agents (1) to (12). In particular, the re-wetting amount in the absorbent body is significantly small in the particulate water-absorbing agents (1) to (20) as compared with the comparative particulate water-absorbing agent (9) having a low SRC (4.83 kPa) and the comparative particulate water-absorbing agent (12) having a low STR. Accordingly, it has been shown that the particulate water-absorbing agents (1) to (20) had high SRC (NP) and thus could absorb a large amount of 0.9% aqueous sodium chloride solution in a short period of time, and had high SRC (4.83 kPa) and thus had excellent liquid retention capacity and reduced re-wetting when the particulate water-absorbing agents (1) to (20) were retained under high pressure after liquid absorption.

As described above, the particulate water-absorbing agent of the present invention has a function of absorbing a large amount of urine in a short period of time, and also has a function of retaining the liquid when a strong pressure is applied from the outside by the user's action or the like after the absorption of the liquid. The function is a function required for a particulate water-absorbing agent used for a sanitary product such as a diaper for adults who urinate a large amount per urination. As a result, it has been found to exhibit the effect of the applicability for producing a sanitary product that has excellent liquid retention properties during actual use and can further reduce re-wetting of absorbed liquid during actual use.

Industrial Applicability

The particulate water-absorbing agent according to an embodiment of the present invention can be widely used in the field of sanitary products such as diapers.

**Claims**

1. A particulate water-absorbing agent, comprising a surface-crosslinked polyacrylic acid (salt)-based water-absorbent resin as a main component, the particulate water-absorbing agent satisfying Formula (A) and Formula (B) set forth below:

$$\text{SRC (NP)} > 50.0 \text{ g/g (A)}$$

$$\text{SRC (4.83 kPa)} > 41.5 \text{ g/g (B)}$$

wherein the SRC (NP) is measured by a method including the following steps (a) to (c):

   (a) obtaining a swollen gel by bringing the particulate water-absorbing agent ($W_0$) [g] into contact with a 0.9% aqueous sodium chloride solution (hereinafter referred to as a "test solution") for 10 minutes with no load applied;
   (b) separating the swollen gel obtained in the step (a) and the test solution;
   (c) measuring a weight ($W_1$) [g] of the swollen gel separated in the step (b), and calculating the SRC (NP) of the particulate water-absorbing agent based on Formula (1) set forth below:

$$\text{SRC (NP) [g/g]} = W_1/W_0 \text{ (1)}$$

   and

   the SRC (4.83 kPa) is measured by the method including the steps (a) to (c) except that, instead of the step (c), steps (c') to (e') described below are performed:

   (c') applying a load of 4.83 kPa to the swollen gel separated in the step (b) for 1 minute;
   (d') removing an exudate exuded from the swollen gel in the step (c'); and
   (e') measuring a weight ($W_2$) [g] of the swollen gel obtained in the step (d'), and calculating the SRC (4.83 kPa) based on Formula (2) set forth below:

$$\text{SRC (4.83 kPa) [g/g]} = W_2/W_0 \text{ (2)}.$$

2. The particulate water-absorbing agent according to claim 1, wherein a value of the SRC (4.83 kPa) in Formula (B) is more than 42 g/g.

3. The particulate water-absorbing agent according to claim 1 or 2, wherein a value of the SRC (NP) in Formula (A) is more than 54.

4. The particulate water-absorbing agent according to any one of claims 1 to 3, wherein a water absorption speed (Vortex method) is 40 seconds or less.

5. The particulate water-absorbing agent according to any one of claims 1 to 4, wherein a surface tension (STR) is 66 mN/m or more.

6. The particulate water-absorbing agent according to any one of claims 1 to 5, wherein a centrifuge retention capacity (CRC) is 35 g/g or more.

7. The particulate water-absorbing agent according to claim 6, wherein a centrifuge retention capacity (CRC) is 40 g/g or more.

8. The particulate water-absorbing agent according to any one of claims 1 to 7, further comprising one or more selected from the group consisting of inorganic colloidal particles, water-insoluble inorganic particles, and a watersoluble polyvalent metal cation-containing compound.

9. An absorbent body, comprising the particulate water-absorbing agent according to any one of claims 1 to 8.

**10.** A sanitary product, comprising the absorbent body according to claim 9.

## INTERNATIONAL SEARCH REPORT

| International application No. |
|---|
| **PCT/JP2024/011819** |

### A. CLASSIFICATION OF SUBJECT MATTER

***B01J 20/26***(2006.01)i; ***A61F 13/53***(2006.01)i; ***B01J 20/28***(2006.01)i
FI:  B01J20/26 D; B01J20/28 Z; A61F13/53 300

According to International Patent Classification (IPC) or to both national classification and IPC

### B. FIELDS SEARCHED

Minimum documentation searched (classification system followed by classification symbols)

B01J20/26; B01J20/28; B01J20/30; C08F20/06; C08F120/06; C08F220/06; C08L33/02-33/12; A61F13/53

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | WO 2021/201177 A1 (NIPPON SHOKUBAI CO., LTD.) 07 October 2021 (2021-10-07) paragraphs [0045]-[0047], [0053]-[0058], [0229]-[0265], [0285], [0286] | 1-10 |
| A | WO 2016/204302 A1 (NIPPON SHOKUBAI CO., LTD.) 22 December 2016 (2016-12-22) paragraphs [0495]-[0500] | 1-10 |
| A | JP 2007-500765 A (KIMBERLY-CLARK WORLDWIDE, INC.) 18 January 2007 (2007-01-18) paragraphs [0079]-[0096], fig. 1 | 1-10 |

☐ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **03 June 2024** | **11 June 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

International application No.

**PCT/JP2024/011819**

| Patent document cited in search report | | | Publication date (day/month/year) | Patent family member(s) | | | Publication date (day/month/year) |
|---|---|---|---|---|---|---|---|
| WO | 2021/201177 | A1 | 07 October 2021 | US | 2023/0144119 | A1 | |
| | | | | paragraphs [0052]-[0055], [0062]-[0066], [0279]-[0315], table 1 | | | |
| | | | | EP | 4130053 | A1 | |
| | | | | CN | 115348897 | A | |
| WO | 2016/204302 | A1 | 22 December 2016 | US | 2018/0298132 | A1 | |
| | | | | paragraphs [0767]-[0776] | | | |
| | | | | US | 2023/0097487 | A1 | |
| | | | | EP | 3312218 | A1 | |
| | | | | CN | 107709415 | A | |
| | | | | KR | 10-2018-0019558 | A | |
| JP | 2007-500765 | A | 18 January 2007 | US | 8269060 | B2 | |
| | | | | fig. 1, column 15, line 7 to column 17, line 37 | | | |
| | | | | WO | 2005/016393 | A1 | |
| | | | | EP | 1654014 | A1 | |
| | | | | TW | 200503661 | A | |
| | | | | KR | 10-2006-0054406 | A | |

Form PCT/ISA/210 (patent family annex) (July 2022)

**REFERENCES CITED IN THE DESCRIPTION**

**Patent documents cited in the description**

- WO 2021201177 A
- US 7638570 B
- US 8269060 B
- WO 2011040530 A
- US 20080161512 A
- WO 2009123197 A
- US 20080194863 A
- US 20050215734 A
- US 6241928 B
- US 7265190 B
- US 4893999 A
- US 2005215734 A

- US 6987151 B
- US 6710141 B
- WO 2011126079 A
- WO 2006100300 A
- WO 2011025012 A
- WO 2011025013 A
- WO 2011111657 A
- US 7183456 B
- WO 97017397 A
- US 6107358 A
- WO 2014034667 A
- WO 2017170605 A

**Non-patent literature cited in the description**

- Japanese Industrial Standards: JIS, Dictionary of Industrial Terms, 2002